(19) Europäisches Patentamt
European Patent Office
Office européen des brevets

(11) EP 1 696 025 B1

(12) EUROPEAN PATENT SPECIFICATION

(45) Date of publication and mention of the grant of the patent:
**21.04.2010 Bulletin 2010/16**

(51) Int Cl.:
***C12N 9/00*** *(2006.01)* *C12N 15/10* *(2006.01)*

(21) Application number: **06009151.9**

(22) Date of filing: **17.07.1996**

(54) **Screening methods for enzymes and enzyme kits**

Screeningverfahren für Enzyme und Enzymkits

Procédes de criblage pour des enzymes et des kits d'enzyme

(84) Designated Contracting States:
**AT BE CH DE DK ES FI FR GB GR IE IT LI LU MC NL PT SE**

(30) Priority: **18.07.1995 US 503606**
**07.12.1995 US 568994**
**03.06.1996 US 657409**

(43) Date of publication of application:
**30.08.2006 Bulletin 2006/35**

(62) Document number(s) of the earlier application(s) in accordance with Art. 76 EPC:
**96925351.7 / 0 839 185**

(73) Proprietor: **Verenium Corporation**
**San Diego, CA 92121 (US)**

(72) Inventor: **Short, Jay. M.**
**Encinitas CA 92024 (US)**

(74) Representative: **Vossius & Partner**
**Siebertstraße 4**
**81675 München (DE)**

(56) References cited:
**JP-A- 2 238 882 US-A- 4 675 285**

- **1991, STRATAGENE , XP002191523 Product Catalogue; "Innovations in Molecular Biology" * page 60 - page 61 ***
- **1994, PROMEGA , XP002191524 Catalog Biological Research Products; "Tools to Explore New Worlds" * page 170 - page 175 ***
- **SAMBROOK J ET AL: "MOLECULAR CLONING. LABORATORY MANUAL. Vol. 2" 1989, COLD SPRING HARBOUR PRESS , NEW YORK , XP002203437 * page 8.50 - page 8.51 ***
- **MCCORMICK M: "SIB SELECTION" METHODS IN ENZYMOLOGY, ACADEMIC PRESS INC, SAN DIEGO, CA, US, vol. 151, 1987, pages 445-449, XP001087654 ISSN: 0076-6879**
- **BARNS SUSAN M ET AL: "Remarkable archaeal diversity detected in a Yellowstone National Park hot spring environment" PROCEEDINGS OF THE NATIONAL ACADEMY OF SCIENCES OF USA, NATIONAL ACADEMY OF SCIENCE, WASHINGTON, DC, US, vol. 91, no. 5, March 1994 (1994-03), pages 1609-1613, XP002139887 ISSN: 0027-8424**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 696 025 B1

Printed by Jouve, 75001 PARIS (FR)

## Description

This invention relates to the field of screening libraries of clones containing microbially derived DNA. Also described are recombinant enzyme expression libraries, recombinant enzyme libraries and kits prepared therefrom which recombinant enzymes are generated from DNA obtained from microorganisms.

Industry has recognized the need for new enzymes for a wide variety of industrial applications. As a result, a variety of microorganisms have been screened to ascertain whether or not such microorganisms have a desired enzyme activity. If such microorganism does have a desired enzyme activity, the enzyme is then recovered from the microorganism.

Naturally occurring assemblages of microorganisms often encompass a bewildering array of physiological and metabolic diversity. In fact, it has been estimated that to date less than one percent of the world's organisms have been cultured. It has been suggested that a large fraction of this diversity thus far has been unrecognized due to difficulties in enriching and isolating microorganisms in pure culture. Therefore, it has been difficult or impossible to identify or isolate valuable enzymes from these samples. These limitations suggest the need for alternative approaches to characterize the physiological and metabolic potential i.e. activities of interest of as-yet uncultivated microorganisms, which to date have been characterized solely by analyses of PCR amplified rRNA gene fragments, clonally recovered from mixed assemblage nucleic acids.

The present invention is defined by the claims.

Also described herein is a novel approach for obtaining enzymes for further use, for example, for packaging into kits for further research. Recombinant enzymes are generated from microorganisms and are classified by various enzyme characteristics. In this manner, the enzymes can be provided as packaged enzyme screening kits, with enzymes in the kit being grouped to have selected enzyme characteristics.

More particularly, a recombinant expression library may be comprised of a multiplicity of clones which are capable of expressing recombinant enzymes. The expression library is produced by recovering DNA from a microorganism, cloning such DNA into an appropriate expression vector which is then used to transfect or transform an appropriate host for expression of a recombinant protein.

Thus, for example, genomic DNA may be recovered from either a culturable or non-culturable organism and employed to produce an appropriate recombinant expression library for subsequent determination of enzyme activity.

Also described is that a recombinant expression library may be prepared without prescreening the organism from which the library is prepared for enzyme activity.

Having prepared a multiplicity of recombinant expression clones from DNA isolated from an organism, the polypeptides expressed by such clones are screened for enzyme activity and specified enzyme characteristics in order to identify and classify the recombinant clones which produce polypeptides having specified enzyme characteristics.

Accordingly the invention provides a process of screening clones having DNA from an uncultivated microorganism for a specified protein, *e.g.* enzyme, activity which process comprises:

screening for a specified protein, *e.g.* enzyme, activity in a library of clones prepared by

(i) recovering DNA from a DNA population derived from at least one uncultivated microorganism; and
(ii) transforming a host with recovered DNA to produce a library of clones which are screened for the specified protein, *e.g.* enzyme, activity.

The library is produced from DNA which is recovered without culturing of an organism, particularly where the DNA is recovered from an environmental sample containing microorganisms which are not or cannot be cultured.

In a preferred embodiment of the invention DNA is ligated into a vector, particularly wherein the vector further comprises expression regulatory sequences which can control and regulate the production of a detectable enzyme activity from the ligated DNA.

The f-factor (or fertility factor) in *E. coli* is a plasmid which effects high frequency transfer of itself during conjugation and less frequent transfer of the bacterial chromosome itself. To archieve and stably propogate large DNA fragments from mixed microbial samples, a particularly preferred embodiment is to use a cloning vector containing an f-factor origin of replication to generate genomic libraries that can be replicated with a high degree of fidelity. When integrated with DNA from a mixed uncultured environmental sample, this makes it possible to achieve large genomic fragments in the form of a stable "environmental DNA library."

Double stranded DNA obtained from the uncultivated DNA population may be selected by:

converting the double stranded genomic DNA into single stranded DNA:

recovering from the converted single stranded DNA single stranded DNA which specifically binds, such as by

hybridization, to a probe DNA sequence: and
converting recovered single stranded DNA to double stranded DNA.

The probe may be directly or indirectly bound to a solid phase by which it is separated from single stranded DNA which is not hybridized or otherwise specifically bound to the probe.

The process can also include releasing single stranded DNA from said probe after recovering said hybridized or otherwise bound single stranded DNA and amplifying the single stranded DNA so released prior to converting it to double stranded DNA.

Also described herein is a process of screening clones having DNA from an uncultivated microorganisms for a specified protein, *e.g*. enzyme, activity which comprises screening for a specified gene cluster protein product activity in the library of clones prepared by: (i) recovering DNA from a DNA population derived from at least one uncultivated microorganism; and (ii) transforming a host with recovered DNA to produce a library of clones with the screens for the specified protein, *e.g*. enzyme, activity. The.library is produced from gene cluster DNA which is recovered without culturing of an organism, particularly where the DNA gene clusters are recovered from an environmental sample containing microorganisms which are not or cannot be cultured.

Alternatively, double-stranded gene cluster DNA obtained from the uncultivated DNA population is selected by converting the double-stranded genomic gene cluster DNA into single-stranded DNA; recovering from the converted single-stranded gene cluster polycistron DNA, single-stranded DNA which specifically binds, such as by hybridization, to a polynucleotide probe sequence; and converting recovered single-stranded gene cluster DNA to double-stranded DNA.

## Brief Description of the Drawings

Figure 1 shows an overview of the procedures used to construct an environmental library from a mixed picoplankton sample as described in Example 3.

Figure 2 is a schematic representation of one embodiment of various tiers of chemical characteristics of an enzyme which may be employed in the present invention as described in Example 4.

Figure 3 is a schematic representation of another embodiment of various tiers of chemical characteristics of an enzyme which may be employed in the present invention as described in Example 4.

Figure 4 is a schematic representation of a further embodiment of various tiers of chemical characteristics of an enzyme which may be employed in the present invention as described in Example 4.

Figure 5 is a schematic representation of a still further embodiment of various tiers of chemical characteristics of an enzyme which may be employed in the present invention as described in Example 4.

Figure 6 shows the pH optima results produced by enzyme ESL-001-01 in the experiments described in Example 5.

Figure 7 shows the temperature optima results produced by enzyme ESL-001-01 in the experiments described in Example 5.

Figure 8 shows the organic solvent tolerance results produced by enzyme ESL-001-01 in the experiments described in Example 5.

## Detailed Description of Preferred Embodiments

The recombinant enzymes described herein are characterized by both physical and chemical characteristics and such chemical characteristics are preferably classified in a tiered manner such that recombinant enzymes having a chemical characteristic in common are then classified by other chemical characteristics which may or may not be more selective or specific chemical characteristic and so on, as hereinafter indicated in more detail.

As hereinabove indicated, the recombinant enzymes are also preferably classified by physical characteristics and one or more tiers of the enzymes which are classified by chemical characteristics may also be classified by physical characteristics or vice versa.

As used herein, the term "chemical characteristic" of a recombinant enzyme refers to the substrate or chemical

functionality upon which the enzyme acts and/or the catalytic reaction performed by the enzyme; e.g., the catalytic reaction may be hydrolysis (hydrolases) and the chemical functionality may be the type of bond upon which the enzyme acts (esterases cleave ester bonds) or may be the particular type of structure upon which the enzyme acts (a glycosidase which acts on glycosidic bonds). Thus, for example, a recombinant enzyme which acts on glycosidic bonds may, for example, be chemically classified in accordance with the tiered system as: Tier 1: hydrolase; Tier 2: acetal bonds: Tier 3: glycosidase.

As used herein, a "physical characteristic" with respect to a recombinant enzyme means a property (other than a chemical reaction) such as pH; temperature stability; optimum temperature for catalytic reaction: organic solvent tolerance: metal ion selectivity: detergent sensitivity, etc.

In a tiered approach for classifying the recombinant enzymes by chemical and/or physical characteristics, the enzymes at one or more of the chemical characteristic tiers may also be classified by one or more physical characteristics and vice versa. It is preferred that The enzymes may be classified by both physical and chemical characteristics, e.g., the individual substrates upon which they act as well as physical characteristics.

Thus, for example, as a representative example of the manner in which a recombinant enzyme may be classified, a recombinant enzyme which is a protease (in this illustration Tier I is hydrolase; Tier 2 is amide (peptide bond) that may be further classified in Tier 3 as to the ultimate site in the amino acid sequence where cleavage occurs: e.g., anion, cation, large hydrophobic, small hydrophobic. Each of the recombinant enzymes which has been classified by the side chain in Tier 3 may also be further classified by physical characteristics of the type hereinabove indicated.

In this manner, it is possible to select from the recombinant library, enzymes which have a specified chemical characteristic in common. e.g., all endopeptidases (which act on internal peptide bonds) and which have a specified physical characteristic in common. e.g., all act optimally at a pH within a specified range.

As hereinabove indicated, a recombinant enzyme library prepared from a microorganism is preferably classified by chemical characteristics in a tiered approach. This may be accomplished by initially testing the recombinant polypeptides generated by the library in a low selectivity screen, e.g., the catalytic reaction performed by the enzyme. This may be conveniently accomplished by screening for one or more of the six IUB classes; Oxidoreductases; transferases; hydrolases: lyases, isomerases, ligases.

The recombinant enzymes which are determined to be positive for one or more of the IUB classes may then be rescreened for a more specific enzyme activity.

Thus, for example, if the recombinant library is screened for hydrolase activity, then those recombinant clones which are positive for hydrolase activity may be rescreened for a more specialized hydrolase activity, i.e., the type of bond on which the hydrolase acts. Thus, for example, the recombinant enzymes which are hydrolases may be rescreened to ascertain those hydrolases which act on one or more specified chemical functionalities, such as: (a) amide (peptide bonds), i.e., proteases; (b) ester bonds, i.e., esterases and lipases; (c) acetals. i.e., glycosidases, etc.

The recombinant enzymes which have been classified by the chemical bond on which they act may then be rescreened to determine a more specialized activity therefor, such as the type of substrate on which they act.

Thus, for example, those recombinant enzymes which have been classified as acting on ester bonds (lipases and esterases) may be rescreened to determine the ability thereof to generate optically active compounds, i.e., the ability to act on specified substrates, such as meso alcohols, meso diacids, chiral alcohols, chiral acids, etc.

For example, the recombinant enzymes which have been classified as acting on acetals may be rescreened to classify such recombinant enzymes by a specific type of substrate upon which they act, e.g., (a) P1 sugar such as glucose, galactose, etc., (b) glucose polymer (exo-, endo- or both), etc. Enzyme Tiers

Thus, as a representative but not limiting example, the following are representative enzyme tiers:

TIER 1. Divisions are based upon the catalytic reaction performed by the enzyme, e.g., hydrolysis, reduction, oxidation, etc. The six 1UB classes will be used: Oxidoreductase. Transferases, Hydrolases, Lyases. Isomerases. Ligases.

TIER 2: Divisions are based upon the chemical functionality undergoing reaction, e.g., esters, amides, phosphate diesters, sulfate mono esters, aldehydes, ketones, alcohols, acetals, ketals, alkanes, olefins, aromatic rings, heteroaromatic rings, molecular oxygen, enols, etc.

Lipases and esterases both cleave the ester bond: the distinction comes in whether the natural substrate is aggregated into a membrane (lipases) of dispersed into solution (esterases)

TIER 3: Divisions and subdivisions are based upon the differences between individual substrate structures which are covalently attached to the functionality undergoing reaction as defined in Tier 2. For example acetal hydrolysis: is the acetal part of glucose or galactose; or is the acetal the $\alpha$ or $\beta$ anomer? These are the types of distinctions made in TIER 3. The divisions based upon substrate specificity are unique to each particular enzyme reaction; there

will be different substrate distinctions depending upon whether the enzyme is, for example, a protease or phosphatase.

TIER 4: Divisions are based on which of the two possible enantiomeric products the enzyme produces. This is a measure of the ability of the enzyme to selectively react with one of the two enantiomers (kinetic resolution), or the ability of the enzyme to react with a *meso* difunctional compound to selectively generate one of the two enantiomeric reaction products.

TIER 5/ORTHOGONAL TIER/PHYSICAL CHARACTER TIER. The fifth tier is orthogonal to the other tiers. It is based on the *physical* properties of the enzymes, rather than the chemical reactions, *per se*: The fifth Tier forms a second dimension with which to classify the enzymes. The Fifth Tier can be applied to any of the other Tiers, but will most often be applied to the Third Tier.

Thus, an expression library may be randomly produced from the DNA of a microorganism, in particular, the genomic DNA or cDNA of the microorganism and the recombinant proteins or polypeptides produced by such expression library are screened to classify the recombinant enzymes by different enzyme characteristics. The recombinant proteins may be screened for one or more particular chemical characteristics and the enzymes identified as having such characteristics are then rescreened for a more specific chemical characteristic and this rescreening may be repeated one or more times. In addition, the recombinant enzymes may also be screened to classify such enzymes by one or more physical characteristics. In this manner, the recombinant enzymes generated from the DNA of a microorganism are classified by both chemical and physical characteristics and it is therefore possible to select recombinant enzymes from one or more different organisms that have one or more common chemical characteristics and/or one or more common physical characteristics. Moreover, since such enzymes are recombinant enzymes, it is possible to produce such enzymes in desired quantities and with a desired purity.

The tiered approach is not limited to a tiered approach in which, for example, the tiers are more restrictive. For example, the tiered approach is also applicable to using a tiered approach in which, for example, the first tier is "wood degrading" enzymes. The second chemical tier could then, for example, be the type of enzyme which is a "wood degrading" enzyme.

Similarly, the first tier or any other tier could be physical characteristics and the next tier could be specified chemical characteristics.

Thus, the described approach is generally applicable to providing recombinant enzymes and recombinant enzyme libraries wherein various enzymes are classified by different chemical and/or physical characteristics.

The microorganisms from which the recombinant libraries may be prepared include prokaryotic microorganisms, such as Eubacteria and Archaebacteria, and lower eukaryotic microorganisms such as fungi, some algae and protozoa. The microorganisms may be cultured microorganisms or uncultured microorganisms obtained from environmental samples and such microorganisms may be extremophiles, such as thermophiles, hyperthermophiles, psychrophiles, psychrotrophs, etc.

Preferably, the library is produced from DNA which is recovered without culturing of an organism, particularly where the DNA is recovered from an environmental sample containing microorganisms which are not or cannot be cultured. Sources of microorganism DNA as a starting material library from which DNA is obtained are particularly contemplated to include environmental samples, such as microbial samples obtained from Arctic and Antarctic ice water or permafrost sources, materials of volcanic origin, materials from soil or plant sources in tropical areas, etc. Thus, for example, genomic DNA may be recovered from either uncultured or non-culturable organism and employed to produce an appropriate library of clones for subsequent determination of enzyme activity.

Bacteria and many eukaryotes have a coordinated mechanism for regulating genes whose products are involved in related processes. The genes are clustered, in structures referred to as "gene clusters," on a single chromosome and are transcribed together under the control of a single regulatory sequence, including a single promoter which initiates transcription of the entire cluster. The gene cluster, the promoter, and additional sequences that function in regulation altogether are referred to as an "operon" and can include up to 20 or more genes, usually from 2 to 6 genes. Thus, a gene cluster is a group of adjacent genes that are either identical or related, usually as to their function.

Some gene families consist of identical members. Clustering is a prerequisite for maintaining identity between genes, although clustered genes are not necessarily identical. Gene clusters range from extremes where a duplication is generated to adjacent related genes to cases where hundreds of identical genes lie in a tandem array. Sometimes no significance is discernable in a repetition of a particular gene. A principal example of this is the expressed duplicate insulin genes in some species, whereas a single insulin gene is adequate in other mammalian species.

It is important to further research gene clusters and the extent to which the full length of the cluster is necessary for the expression of the proteins resulting therefrom. Further, gene clusters undergo continual reorganization and, thus, the ability to create heterogeneous libraries of gene clusters from, for example, bacterial or other prokaryote sources is

valuable in determining sources of novel proteins, particularly including proteins, *e.g.* enzymes, such as, for example, the polyketide synthases that are responsible for the synthesis of polyketides having a vast array of useful activities. Other types of proteins that are the product(s) of gene clusters are also contemplated, including, for example, antibiotics, antivirals, antitumor agents and regulatory proteins, such as insulin.

Polyketides are molcules which are an extremely rich source of bioactivities, including antibiotics (such as tetracyclines and erythromycin), anti-cancer agents (daunomycin), immunosuppressants (FK506 and rapamycin), and veterinary products (monensin). Many polyketides (produced by polyketide synthases) are valuable as therapeutic agents. Polyketide synthases are multifunctional enzymes that catalyze the biosynthesis of a huge variety of carbon chains differing in length and patterns of functionality and cyclization. Polyketide synthase genes fall into gene clusters and at least one type (designated type 1) of polyketide synthases have large size genes and enzymes, complicating genetic manipulation and *in vitro* studies of these genes/proteins.

The ability to select and combine desired components from a library of polyketide and postpolyketide biosynthesis genes for generation of novel polyketides for study is appealing. Using the method(s) described herein facilitates the cloning of novel polyketide synthases, particularly when one uses the f-factor based vectors, which facilitate cloning of gene clusters.

Preferably, the gene cluster DNA is ligated into a vector, particularly wherein a vector further comprises expression regulatory sequences which can control and regulate the production of a detectable protein or protein-related array activity from the ligated gene clusters. Use of vectors which have an exceptionally large capacity for exogenous DNA introduction are particularly appropriate for use with such gene clusters and are described by way of example herein to include the f-factor (or fertility factor) of *E. coli.* This f-factor of *E. coli* is a plasmid which affect highfrequency transfer of itself during conjugation and is ideal to achieve and stably propagate large DNA-fragments, such as gene clusters from mixed microbial samples.

The term "derived" or "isolated" means that material is removed from its original environment (*e.g.*. the natural environment if it is naturally occurring). For example, a naturally-occurring polynucleotide or polypeptide present in a living animal is not isolated, but the same polynucleotide or polypeptide separated from some or all of the coexisting materials in the natural system, is isolated.

As hereinabove indicated, the expression library may be produced from environmental samples in which case DNA may be recovered without culturing of an organism or the DNA may be recovered from a cultured organism.

In preparing the expression library genomic DNA may be recovered from either a cultured organism or an environmental sample (for example, soil) by various procedures: The recovered or isolated DNA is then fragmented into a size suitable for producing an expression library and for providing a reasonable probability that desired genes will be expressed and screened without the necessity of screening an excessive number of clones. Thus, for example, if the average genome fragment produced by shearing is 4.5 kbp, for a 1.8Mbp genome about 2000 clones should be screened to achieve about a 90% probability of obtaining a particular gene. In some cases, in particular where the DNA is recovered without culturing, the DNA is amplified (for example by PCR) after shearing.

The sized DNA is cloned into an appropriate expression vector and transformed into an appropriate host, preferably a bacterial host and in particular *E. coli.* Although *E. coli* is preferred, a wide variety of other hosts may be used for producing an expression library.

The expression vector which is used is preferably one which includes a promoter which is known to function in the selected host in case the native genomic promoter does not function in the host.

As representative examples of expression vectors which may be used for preparing an expression library, there may be mentioned phage, plasmids, phagemids cosmids, phosmids, bacterial artificial chromosomes, P1-based artificial chromosomes, yeast artificial chromosomes, and any other vectors specific for specific hosts of interest (such as bacillus, aspergillus, yeast, etc.) The vector may also include a tag of a type known in the an to facilitate purification.

The following outlines a general procedure for producing expression libraries from both culturable and non-culturable organisms.

**CULTURABLE ORGANISMS**

Obtain Biomass
DNA Isolation (CTAB)
Shear DNA (25 gauge needle)
Blunt DNA (Mung Bean Nuclease)
Methylate (*Eco* RI Methylase)
Ligate to *Eco* RI linkers (GGAATTCC)
Cut back linkers (*Eco* RI Restriction Endonuclease)

Size Fractionate (Sucrose Gradient)
Ligate to lambda vector (Lambda ZAP II and gt11)
Package (*in vitro* lambda packaging extract)
Plate on *E. coli* host and amplify

## UNCULTURABLE ORGANISMS

Obtain cells
Isolate DNA (Various Methods)
Blunt DNA (Mung Bean Nuclease)
Ligate to adaptor containing a *Not* I site and conjugated to magnetic beads
Ligate unconjugated adaptor to the other end of the DNA
Amplify DNA in a reaction which allows for high fidelity, and uses adaptor sequences as primers
Cut DNA with *Not* I
Size fractionate (Sucrose Gradient or Sephacryl Column)
Ligate to lambda vector (Lambda ZAP II and gt11)
Package (*in vitro* lambda packaging extract)
Plate on *E. coli* host and amplify

The probe DNA used for selectively recovering DNA of interest from the DNA derived from the at least one uncultured microorganism can be a full-length coding region sequence or a partial coding region sequence of DNA for an enzyme of known activity, a phylogenetic marker or other identified DNA sequence. The original DNA library can be preferably probed using mixtures of probes comprising at least a portion of the DNA sequence encoding the specified activity. These probes or probe libraries are preferably single-stranded and the microbial DNA which is probed has preferably been converted into single-stranded form. The probes that are particularly suitable are those derived from DNA encoding enzymes having an activity similar or identical to the specified enzyme activity which is to be screened.

The probe DNA should be at least about 10 bases and preferably at least 15 bases. In one embodiment, the entire coding region may be employed as a probe. Conditions for the hybridization in which DNA is selectively isolated by the use of at least one DNA probe will be designed to provide a hybridization stringency of at least about 50% sequence identity, more particularly a stringency providing for a sequence identity of at least about 70%.

Hybridization techniques for probing a microbial DNA library to isolate DNA of potential interest are well known in the art and any of those which are described in the literature are suitable for use herein, particularly those which use a solid phase-bound, directly or indirectly bound, probe DNA for ease in separation from the remainder of the DNA derived from the microorganisms

Preferably the probe DNA is "labeled" with one partner of a specific binding pair (*i.e*. a ligand) and the other partner of the pair is bound to a solid matrix to provide ease of separation of target from its source. The ligand and specific binding partner can be selected from, in either orientation, the following: (1) an antigen or hapten and an antibody or specific binding fragment thereof: (2) biotin or iminobiotin and avidin or streptavidin; (3) a sugar and a lectin specific therefor; (4) an enzyme and an inhibitor therefor: (5) an apoenzyme and cofactor: (6) complementary homopolymeric oligonucleotides; and (7) a hormone and a receptor therefor. The solid phase is preferably selected from: (1) a glass or polymeric surface; (2) a packed column of polymeric beads; and (3) magnetic or paramagnetic particles.

The library of clones prepared as described above can be screened directly for a desired, *e.g*. enzymatic, activity without the need for culture expansion, amplification or other supplementary procedures. However, in one preferred embodiment, it is considered desirable to amplify the DNA recovered from the individual clones such as by PCR.

Further, it is optional but desirable to perform an amplification of the target DNA that has been isolated. In this embodiment the selectively isolated DNA is separated from the probe DNA after isolation. It is then amplified before being used to transform hosts. The double stranded DNA selected to include as at least a portion thereof a predetermined DNA sequence can be rendered single stranded, subjected to amplification and reannealed to provide amplified numbers of selected double stranded DNA. Numerous amplification methodologies are now well known in the art.

The selected DNA is then used for preparing a library for screening by transforming a suitable organism. Hosts, particularly those specifically identified herein as preferred, are transformed by artificial introduction of the vectors containing the target DNA by inoculation under conditions conducive for such transformation.

As representative examples of expression vectors which may be used there may be mentioned viral particles, baculovirus, phage, plasmids, phagemids, cosmids, phosmids, bacterial artificial chromosomes, viral DNA (*e.g*. vaccinia, adenovirus, foul pox virus, pseudorabies and derivatives of SV40), P1-based artificial chromosomes, yeast plasmids, yeast artificial chromosomes, and any other vectors specific for specific hosts of interest (such as bacillus, aspergillus,

yeast, etc.) Thus, for example, the DNA may be included in any one of a variety of expression vectors for expressing a polypeptide. Such vectors include chromosomal, nonchromosomal and synthetic DNA sequences. Large numbers of suitable vectors are known to those of skill in the art, and are commercially available. The following vectors are provided by way of example: Bacterial: pQE70, pQE60, pQE-9 (Qiagen), psiX174, pBluescript SK, pBluescript KS, pNH8A, pNH16a, pNH18A, pNH46A (Stratagene); pTRC99a, pKK223-3, pKK233-3, pDR540, pRIT5 (Pharmacia); Eukaryotic: pWLNEO, pSV2CAT, pOG44. pXT1, pSG (Stratagene) pSVK3, pBPV, pMSG. pSVL (Pharmacia). However, any other plasmid or vector may be used as long as they are replicable and viable in the host.

A particularly preferred type of vector for use in the present invention contains an f-factor origin of replication. The f-factor (or fertility factor) in *E. coli* is a plasmid which effects high frequency transfer of itself during conjugation and less frequent transfer of the bacterial chromosome itself. A particularly preferred embodiment is to use cloning vectors, referred to as "fosmids" or bacterial artificial chromosome (BAC) vectors. These are derived from the *E. coli* f-factor and are able to stably integrate large segments of genomic DNA. When integrated with DNA from a mixed uncultured environmental sample, this makes it possible to achieve large genomic fragments in the form of a stable "environmemental DNA library."

The DNA derived from a microorganism(s) may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence (s) (promoter) to direct mRNA synthesis. Particular named bacterial promoters include lacI, lacZ, T3, T7, gpt, lambda $P_R$, $P_L$ and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. The expression vector also contains a ribosome binding site for translation initiation and a transcription terminator. The vector may also include appropriate sequences for amplifying expression. Promoter regions can be selected from any desired gene using CAT (chloramphenicol transferase) vectors or other vectors with selectable markers.

In addition, the expression vectors.preferably contain one or more selectable marker genes to provide a phenotypic trait for selection of transformed host cells such as dihydrofolate reductase or neomycin resistance for eukaryotic cell culture, or such as tetracycline or ampicillin resistance in *E. coli*.

Generally, recombinant expression vectors will include origins of replication and selectable markers permitting transformation of the host cell. *e.g.,* the ampicillin resistance gene of *E. coli* and *S. cerevisiae* TRP1 gene, and a promoter derived from a highly-expressed gene to direct transcription of a downstream structural sequence Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium.

The DNA selected and isolated as hereinabove described is introduced into a suitable host to prepare a library which is screened for the desired enzyme activity. The selected DNA is preferably already in a vector which includes appropriate control sequences whereby selected DNA which encodes for an enzyme may be expressed, for detection of the desired activity. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by transformation, calcium phosphate transfection, DEAE-Dextran mediated transfection, DMSO or electroporation (Davis, L., Dibner, M., Battey, I., Basic Methods in Molecular Biology, (1986)).

As representative examples of appropriate hosts, there may be mentioned: bacterial cells, such as *E. coli. Bacillus*, *Streptomyces, Salmonella typhimurium*; fungal cells, such as yeast: insect cells such as *Drosophila* S2 and *Spodoptera Sf9*: animal cells such as CHO. COS or Bowes melanoma: adenoviruses; plant cells, *etc.* The selection of an appropriate host is deemed to be within the scope of those skilled in the art from the teachings herein.

Host cells are genetically engineered (transduced or transformed or transfected) with the vectors. The engineered host cells can be cultured in conventional nutrient media modified as appropriate for activating promoters, selecting transformants or amplifying genes. The culture conditions, such as temperature, pH and the like, are those previously used with the host cell selected for expression, and will be apparent to the ordinarily skilled artisan.

The recombinant enzymes in the library which are classified as described herein may or may not be sequenced and may or may not be in a purified form. Thus, in accordance with the present invention, it is possible to classify one or more of the recombinant enzymes before or after obtaining the sequence of the enzyme or before or after purifying the enzyme to essential homogeneity.

The screening for chemical characteristics may be effected on individual expression clones or may be initially effected on a mixture of expression clones to ascertain whether or not the mixture has one or more specified enzyme activities. If the mixture has a specified enzyme activity, then the individual clones may be rescreened for such enzyme activity or for a more specific activity. Thus, for example, if a clone mixture has hydrolase activity, then the individual

clones may be recovered and screened to determine which of such clones has hydrolase activity.

Described are also enzyme kits for use in further screening and/or research. Thus, a reagent package or kit may be prepared by placing in the kit or package, e.g., in suitable containers, at least three different recombinant enzymes with each of the at least three different recombinant enzymes having at least two enzyme characteristics in common. One common characteristic may be a chemical characteristic or property and the other common characteristic is a physical characteristic or property: however, it is possible to prepare kits which have two or more chemical characteristics or properties in common and no physical characteristics or property in common and vice versa.

Since, it is possible to provide a recombinant enzyme library from one or more microorganisms which is classified by a multiplicity of chemical and/or physical properties, a variety of enzyme kits or packages can be prepared having a variety of selected chemical and/or physical characteristics which can be formulated to contain three or more recombinant enzymes in which at least three and preferably all of the recombinant enzymes have in common at least one chemical characteristic and have in common at least one physical characteristic. The kit should contain an appropriate label specifying such common characteristics.

At least three recombinant enzymes in the kit may have in common the most specific chemical characteristic specified on the label. The term "label" is used in its broadest sense and includes package inserts or literature associated or distributed in conjunction with the kit or package. Thus, for example, if the kit is labeled for a specific substrate (one of the Tier 3 examples above), then for example, at least three of the enzymes in the kit would act on such substrate.

The kits may contain more than three enzymes, for example, five, six or more enzymes and in a preferred embodiment at least three or a majority and in some cases all of the recombinant enzymes in the kit may have at least two enzyme properties or characteristics in common, as hereinabove described.

The recombinant enzymes in the kits may have two or more enzymes in a single container or individual enzymes in individual containers or various combinations thereof.

The library may be screened for a specified enzyme activity by procedures known in the art. For example, the enzyme activity may be screened for one or more of the six IUB classes: oxidoreductases, transferases, hydrolases, lyases, isomerases and ligases. The recombinant enzymes which are determined to be positive for one or more of the IUB classes may then be rescreened for a more specific enzyme activity.

Alternatively, the library may be screened for a more specialized enzyme activity. For example, instead of generically screening for hydrolase activity, the library may be screened for a more specialized activity. *i.e*. the type of bond on which the hydrolase acts. Thus, for example, the library may be screened to ascertain those hydrolases which act on one or more specified chemical functionalities, such as: (a) amide (peptide bonds), *i.e*. proteases: (b) ester bonds, *i.e*. esterases and lipases: (c) acetals, *i.e*., glycosidases *etc.*

The clones which are identified as having the specified enzyme activity may then be sequenced to identify the DNA sequence encoding an enzyme having the specified activity. Thus, in accordance with the present invention it is possible to isolate and identify: (i) DNA encoding an enzyme having a specified enzyme activity, (ii) enzymes having such activity (inlcuding the amino acid sequence thereof) and (iii) produce recombinant enzymes having such activity.

The screening for enzyme activity may be effected on individual expression clones or may be initially effected on a mixture of expression clones to ascertain whether or not the mixture has one or more specified enzyme activities. If the mixture has a specified enzyme activity, then the individual clones may be rescreened for such enzyme activity or for a more specific activity. Thus, for example, if a clone mixture has hydrolase activity, then the individual clones may be recovered and screened to determine which of such clones has hydrolase activity.

The expression libraries may be screened for one or more selected chemical characteristics. Selected representative chemical characteristics are described below but such characteristics do not limit the present invention. Moreover, the expression libraries may be screened for some or all of the characteristics. Thus, some of the chemical characteristics specified herein may be determined in all of the libraries, none of the libraries or in only some of the libraries.

The recombinant enzymes may also be tested and classified by physical properties. For example, the recombinant enzymes may be classified by physical properties such as follows:

pH optima

$<3$

3-6

6-9

9-12

$> 12$

temperature optima
> 90˚C
75-90˚C
60-75˚C
45-60˚C
30-45˚C
15-30˚C
0-15˚C

temperature stability
half-life at:
90˚C
75˚C
60˚C
45˚C

organic solvent tolerance
water miscible
(DMF)
90%
75%
45%
30%
water immiscible
hexane
toluene

metal ion selectivity
EDTA - 10 mM
$Ca^{+2}$ - 1 mM
$Mg^{+2}$ - 100 μM
$Mn^{+2}$ - 10 μM
$Co^{+3}$ - 10 μM

detergent sensitivity
neutral (triton)
anionic (deoxycholate)
cationic (CHAPS)

The recombinant enzymes of the described libraries and kits may be used for a variety of purposes. The described approach by providing a plurality of recombinant enzymes classified by a plurality of different enzyme characteristics permits rapid screening of enzymes for a variety of applications.
Thus, for example, the described approach permits assembly of enzyme kits which contain a plurality of enzymes which are capable of operating on a specific bond or a specific substrate at specified conditions to thereby enable screening of enzymes for a variety of applications. As representative examples of such applications, there may be mentioned:

**1. Lipase/Esterase**

a. Enantioselective hydrolysis of esters (lipids)/ thioesters

1) Resolution of racemic mixtures
2) Synthesis of optically active acids or alcohols from *meso*-diesters

b. Selective syntheses

1) Regiospecific hydrolysis of carbohydrate esters
2) Selective hydrolysis of cyclic secondary alcohols

c. Synthesis of optically active esters, lactones, acids, alcohols

1) Transesterification of activated/nonactivated esters
2) Interesterification
3) Optically active lactones from hydroxyesters
4) Regio- and enantioselective ring opening of anhydrides

d. Detergents
e. Fat/Oil conversion
f. Cheese ripening

**2. Protease**

a. Ester/amide synthesis
b. Peptide synthesis
c. Resolution of racemic mixtures of amino acid esters
d. Synthesis of non-natural amino acids
e. Detergents/protein hydrolysis

**3. Glycosidase/Glycosyl transferase**

a. Sugar/polymer synthesis
b. Cleavage of glycosidic linkages to form mono, di-and oligosaccharides
c. Synthesis of complex oligosaccharides
d. Glycoside synthesis using UDP-galactosyl transferase
e. Transglycosylation of disaccharides, glycosyl fluorides, aryl galactosides
f. Glycosyl transfer in oligosaccharide synthesis
g. Diastereoselective cleavage of β-glucosylsulfoxides
h. Asymmetric glycosylations
i. Food processing
j. Paper processing

**4. Phosphatase/Kinase**

a. Synthesis/hydrolysis of phosphate esters

1) Regio-, enantioselective phosphorylation
2) Introduction of phosphate esters

3) Synthesize phospholipid precursors
4) Controlled polynucleotide synthesis

b. Activate biological molecule
c. Selective phosphate bond formation without protecting groups

**5. Mono/Dioxygenase**

a. Direct oxyfunctionalization of unactivated organic substrates
b. Hydroxylation of alkane, aromatics, steroids
c. Epoxidation of alkenes
d. Enantioselective sulphoxidation
e. Regio- and stereoselective Bayer-Villiger oxidations

**6. Haloperoxidase**

a. Oxidative addition of halide ion to nucleophilic sites
b. Addition of hypohalous acids to olefinic bonds
c. Ring cleavage of cyclopropanes
d. Activated aromatic substrates converted to *ortho* and *para* derivatives
e. 1.3 diketones converted to 2-halo-derivatives .
f. Heteroatom oxidation of sulfur and nitrogen containing substrates
g. Oxidation of enol acetates, alkynes and activated aromatic rings

**7. Lignin peroxidase/Diarylpropane peroxidase**

a. Oxidative cleavage of C-C bonds
b. Oxidation of benzylic alcohols to aldehydes
c. Hydroxylation of benzylic carbons
d. Phenol dimerization
e. Hydroxylation of double bonds to form diols
f. Cleavage of lignin aldehydes

**8. Epoxide hydrolase**

a. Synthesis of enantiomerically pure bioactive compounds
b. Regio- and enantioselective hydrolysis of epoxide
c. Aromatic and olefinic epoxidation by monooxygenases to form epoxides
d. Resolution of racemic epoxides
e. Hydrolysis of steroid epoxides

**9. Nitrile hydratase/nitrilase**

a. Hydrolysis of aliphatic nitriles to carboxamides
b. Hydrolysis of aromatic, heterocyclic, unsaturated aliphatic nitriles to corresponding acids
c. Hydrolysis of acrylonitrile
d. Production of aromatic and carboxamides, carboxylic acids (nicotinamide, picolinamide, isonicotinamide)
e. Regioselective hydrolysis of acrylic dinitrile

f. α-amino acids from α-hydroxynitriles

**10. Transaminase**

a. Transfer of amino groups into oxo-acids

**11. Amidase/Acylase**

a. Hydrolysis of amides, amidines, and other C-N bonds
b. Non-natural amino acid resolution and synthesis

The invention will be further described with reference to the following examples; however, the scope of the present invention is not to be limited thereby. Unless otherwise specified, all parts are by weight.

**Example 1**

**Production of Expression Library**

The following describes a representative procedure for preparing an expression library for screening by the tiered approach of the present invention.

One gram of *Thermococcus* GU5L5 cell pellet was lysed and the DNA isolated by literature procedures (Current Protocols in Molecular Biology, 2.4.1, 1987). Approximately 100μg of the isolated DNA was resuspended in TE buffer and vigorously passed through a 25 gauge double-hubbed needle until the sheared fragments were in the size range of 0.5-10.0 Kb (3.0 Kb average). The DNA ends were "polished" or blunted with Mung Bean Nuclease (300 units, 37˚C, 15 minutes), and EcoRI restriction sites in the target DNA protected with EcoRI Methylase (200 units, 37˚C, 1 hour). EcoRI linkers [GGAATTCC] were ligated to the blunted/protected DNA using 10 pmole ends of linkers to lpmole end of target DNA. The linkers were cut back with EcoRI restriction endonuclease (200 units, 37˚C, 1.5 hours) and the DNA size fractionated by sucrose gradient (Maniatis, T., Fritsch, E.F., and Sambrook, J., Molecular Cloning, Cold Spring Harbor Press, New York, 1982). The prepared target DNA was ligated to the Lambda ZAP® II vector (Stratagene), packaged using in vitro lambda packaging extracts and grown on XL1-Blue MRF' *E. coli* strain according to the manufacturer. The pBluescript® phagemids were excised from the lambda library, and grown in *E. coli* DH10B F' kan, according to the method of Hay and Short (Hay and Short. J. Strategies, 5: 16. 1992). The resulting colonies were picked with sterile toothpicks and used to singly inoculate each of the wells of 11 96-well microtiter plates (1056 clones in all). The wells contained 250 μL of LB media with 100 μg/mL ampicillin, 80 μg/mL methicillin, and 10% v/v glycerol (LB Amp/Meth, glycerol). The cells were grown overnight at 37˚C without shaking. This constituted generation of the "Source Library"; each well of the Source Library thus contained a stock culture of *E. coli* cells, each of which contained a pBluescript phagemid with a unique DNA insert.

**Example 2**

**Preparation of a Mammalian DNA Library**

The following outlines the procedures used to generate a gene library from a sample of the exterior surface of a whale bone found at 1240 meters depth in the Santa Catalina Basin during a dive expedition.

**Isolate DNA.**

IsoQuick Procedure as per manufacturer's instructions.

**Shear DNA**

1. Vigorously push and pull DNA through a 25G double-hub needle and 1-cc syringes about 500 times.
2. Check a small amount (0.5 μg) on a 0.8% agarose gel to make sure the majority of the DNA is in the desired size range (about 3-6 kb).

**Blunt DNA**

1. Add:

| | |
|---|---|
| $H_2O$ | to a final volume of 405 µl |
| 45 µl | 10X Mung Bean Buffer |
| 2.0 µl | Mung Bean Nuclease (150 u/µl) |

2. Incubate 37˚C, 15 minutes.
3. Phenol/chloroform extract once.
4. Chloroform extract once.
5. Add 1 ml ice cold ethanol to precipitate.
6. Place on ice for 10 minutes.
7. Spin in microfuge, high speed, 30 minutes.
8. Wash with 1 ml 70% ethanol.
9. Spin in microfuge, high speed, 10 minutes and dry.

**Methylate DNA**

1. Gently resuspend DNA in 26 µl TE.
2. Add:

| | |
|---|---|
| 4.0 µl | 10X *EcoR* I Methylase Buffer |
| 0.5 µl | SAM (32 mM) |
| 5.0 µl | *EcoR* I Methylase (40 u/µl) |

3. Incubate 37˚. 1 hour.

**Insure Blunt Ends**

1. Add to the methylation reaction:

| | |
|---|---|
| 5.0 µl | 100 mM $MgCl_2$ |
| 8.0 µl | dNTP mix (2.5 mM of each dGTP, dATP, dTTP. dCTP) |
| 4.0 µl | Klenow (5 u/µl) |

2. Incubate 12˚C. 30 minutes.
3. Add 450 µl 1X STE.
4. Phenol/chloroform extract once.
5. Chloroform extract once.
6. Add I ml ice cold ethanol to precipitate and place on ice for 10 minutes.
7. Spin in microfuge, high speed. 30 minutes.
8. Wash with 1 ml 70% ethanol.
9. Spin in microfuge, high speed. 10 minutes and dry.

**Linker Ligation**

1. Gently resuspend DNA in 7 µl Tris-EDTA (TE).
2. Add:

| | |
|---|---|
| 14 µl | Phosphorylated *EcoR* I linkers (200 ng/µl) |
| 3.0 µl | 10X Ligation Buffer |
| 3.0 µl | 10 mM rATP |
| 3.0 µl | T4 DNA Ligase (4Wu/µl) |

3. Incubate 4˚C. overnight.

**EcoRI Cutback**

1. Heat kill ligation reaction 68˚C. 10 minutes.
2. Add:
   - 237.9 µl $H_2O$
   - 30 µl 10X *Eco*R I Buffer
   - 2.1 µl *EcoR* I Restriction Enzyme (100 u/µl)
3. Incubate 37˚C, 1.5 hours.
4. Add 1.5 µl 0.5 M EDTA.
5. Place on ice.

**Sucrose Gradient (2.2 ml) Size Fractionation**

1. Heat sample to 65˚C. 10 minutes.
2. Gently load on 2.2 ml sucrose gradient.
3. Spin in mini-ultracentrifuge, 45K. 20˚C. 4 hours (no brake).
4. Collect fractions by puncturing the bottom of the gradient tube with a 20G needle and allowing the sucrose to flow through the needle. Collect the first 20 drops in a Falcon 2059 tube then collect 10 1-drop fractions (labelled 1 -10). Each drop is about 60 µl in volume.
5. Run 5 µl of each fraction on a 0.8% agarose gel to check the size.
6. Pool fractions 1-4 (-10-1.5 kb) and in a separate tube, pool fractions 5-7 (about 5-0.5 kb).
7. Add 1 ml ice cold ethanol to precipitate and place on ice for 10 minutes.
8. Spin in microfuse, high speed, 30 minutes.
9. Wash with 1 ml 70% ethanol.
10. Spin in microfuge, high speed, 10 minutes and dry.
11. Resuspend each in 10 µl TE buffer.

**Test Ligation to Lambda Arms**

1. Plate assay to get an approximate concentration. Spot 0.5 µl of the sample on agarose containing ethidium bromide along with standards (DNA samples of known concentration). View in UV light and estimate concentration compared to the standards. Fraction 1-4 = > 1.0 µg/µl. Fraction 5-7 = 500 ng/µl.
2. Prepare the following ligation reactions (5 µl reactions) and incubate 4˚C. overnight:

| Sample | $H_2O$ | 10X Ligase Buffer | 10mM rATP | Lambda arms (gt11 and ZAP) | Insert DNA | T4 DNA Ligase (4 Wu/µ) |
|---|---|---|---|---|---|---|
| Fraction 1-4 | 0.5 µl | 0.5 µl | 0.5 µl | 10 µl | 2.0 µl | 0.5 µl |
| Fraction 5-7 | 0.5 µl | 0.5 µl | 0.5 µl | 1.0 µl | 2.0 µl | 0.5 µl |

**Test Package and Plate**

1. Package the ligation reactions following manufacturer's protocol. Package 2.5 µl per packaging extract (2 extracts per ligation).
2. Stop packaging reactions with 500 µl SM buffer and pool packaging that came from the same ligation.
3. Titer 1.0 µl of each on appropriate host ($OD_{600}$ = 1.0) [XLI-Blue MRF for ZAP and Y1088 for gt11]
   Add 200 ml host (in mM $MgSO_4$) to Falcon 2059 tubes
   Inoculate with 1 ml packaged phage
   Incubate 37 ˚C, 15 minutes
   Add about 3 ml 48˚C top agar
      [50 ml stock containing 150 ml IPTG (0.5M) and

(continued)

**Test Package and Plate**

300 μl X-GAL (350 mg/ml)]

Plate on 100mm plates and incubate 37˚C, overnight.

4. Efficiency results:

gt11: 1.7 x $10^4$ recombinants with 95 % background

ZAP II: 4.2 x $10^4$ recombinants with 66% background

Contaminants in the DNA sample may have inhibited the enzymatic reactions, though the sucrose gradient and organic extractions may have removed them. Since the DNA sample was precious, an effort was made to "fix" the ends for cloning:

**Re-Blunt DNA**

1. Pool all left over DNA that was not listed to the lambda arms (Fractions 1-7) and add $H_2O$ to a final volume of 12 μl. Then add:

| | |
|---|---|
| 143 μl | $H_2O$ |
| 20 μl | 10X Buffer 2 (from Stratagene's cDNA Synthesis Kit) |
| 23 μl | Blunting dNTP (from Stratagene's cDNA Synthesis Kit) |
| 2.0 μl | Pfu (from Straiagene"s cDNA Synthesis Kit) |

2. Incubate 72˚C, 30 minutes.
3. Phenol/chloroform extract once.
4. Chloroform extract once.
5. Add 20 μL 3M NaOAc and 400 μl ice cold ethanol to precipitate:
6. Place at -20˚C. overnight.
7. Spin in microfuge, high speed, 30 minutes.
8. Wash with 1 ml 70% ethanol.
9. Spin in microfuge, high speed, 10 minutes and dry.

**(Do NOT Methylate DNA since it was already methylated in the first round of processing)**

**Adaptor Ligation**

1. Gently resuspend DNA in 8 μl EcoR I adaptors (from Stratagene's cDNA Synthesis Kit).
2. Add:

| | |
|---|---|
| 1.0 μl | 10X Ligation Buffer |
| 1.0 μl | 10 mM rATP |
| 1.0 μl | T4 DNA Ligase (4Wu/μl) |

3. Incubate 4˚C. 2 days.

**(Do NOT cutback since using ADAPTORS this time. Instead, need to phospherylate)**

**Phosphorylate Adaptors**

1. Heat kill ligation reaction 70˚C, 30 minutes.

Add:

| | |
|---|---|
| 1.0 μl | 10X Ligation Buffer |
| 2.0 μl | 10mM rATF |
| 6.0 μl | $H_2O$ |
| 1.0 μl | PNK (from Stratagene's cDNA Synthesis Kit). |

3. Incubate 37˚C, 30 minutes.
4. Add 31 μl $H_2O$ and 5 μl 10X STE.
5. Size fractionate on a Sephacryl S-500 spin column (pool fractions 1-3).
6. Phenol/chlocoform extract once.

(continued)

**Phosphorylate Adaptors**

7. Chloroform extract once.
8. Add ice cold ethanol to precipitate.
9. Place on ice, 10 minutes.
10. Spin in microfuge, high speed, 30 minutes.
11. Wash with 1 ml 70% ethanol.
12. Spin in microfuge, high speed. 10 minutes and dry.
13. Resuspend in 10.5 μl TE buffer.

**Do not plate assay. Instead, ligate directly to arms as above except use 2.5 μl of** DNA and no water.

**Package and titer as above.**

Efficiency results:

| | |
|---|---|
| *gt*11: | 2.5 x $10^6$ recombinants with 2.5% background |
| ZAP II: | 9.6 x $10^5$ recombinants with 0% background |

**Amplification of Libraries (5.0 x $10^5$ recombinants from each library)**

1. Add 3.0 ml host cells ($OD_{660}$= 1.0) to two 50 ml conical tube.
2. Inoculate with 2.5 X $10^5$ pfu per conical tube.
3. Incubate 37˚C, 20 minutes.
4. Add top agar to each tube to a final volume of 45 ml.
5. Plate the tube across five 150 mm plates.
6. Incubate 37˚C, 6-8 hours or until plaques are about pin-head in size.
7. Overlay with 8-10 ml SM Buffer and place at 4˚C overnight (with gentle rocking if possible).

**Harvest Phage**

1. Recover phage suspension by pouring the SM buffer off each plate into a 50-ml conical tube.
2. Add 3 ml chloroform, shake vigorously and incubate at room temperature, 15 minutes.
3. Centrifuge at 2K rpm, 10 minutes to remove cell debris.
4. Pour supernatant into a sterile flask. add 500 μl chloroform.
5. Store at 4˚C.

**Titer Amplified Library**

1. Make serial dilutions:
   $10^{-5}$ = I μl amplified phage in 1 ml SM Buffer
   $10^{-6}$ = 1 μl of the $10^{-3}$ dilution in I ml SM Buffer
2. Add 200 μl host (in 10 mM $MgSO_4$) to two tubes.
3. Inoculate one with 10 μl $10^{-6}$ dilution ($10^{-5}$).
4. Inoculate the other with 1 μl $10^{-6}$ dilution ($10^{-6}$).
5. Incubate 37˚C, 15 minutes.
6. Add about 3 ml 48˚C top agar.
   [50 ml stock containing 150 μl IPTG (0.5M) and 375 μl X-GAL (350 mg/ml)]
7. Plate on 100 mm plates and incubate 37˚C, overnight.
8. Results:

(continued)

| Titer Amplified Library | | |
|---|---|---|
| | gt11: | $1.7 \times 10^{11}$/ml |
| | ZAP II: | $2.0 \times 10^{10}$/ml |
| **Excise the ZAP II library to create the pBluescript library.** | | |

**Example 3**

**Preparation of an Uncultivated Prokaryotic DNA Library**

Figure 1 shows an overview of the procedures used to construct an environmental library from a mixed picoplankton sample. The goal was to construct a stable, large insert DNA library representing picoplankton genomic DNA.

**Cell collection and preparation of DNA.** Agarose plugs containing concentrated picoplankton cells were prepared from samples collected on an oceanographic cruise from Newport, Oregon to Honolulu, Hawaii. Seawater (30 liters) was collected in Niskin bottles, screened through 10 μm Nitex, and concentrated by hollow fiber filtration (Amicon DC10) through 30,000 MW cutoff polysulfone filters. The concentrated bacterioplankton cells were collected on a 0.22 μm, 47 mm Durapore filter, and resuspended in I ml of 2X STE buffer (1M NaCl, 0.1M EDTA, 10 mM Tris, pH 8.0) to a final density of approximately 1 X $10^{10}$ cells per ml. The cell suspension was mixed with one volume of 1 % molten Seaplaque LMP agarose (FMC) cooled to 40˚C, and then immediately drawn into a 1 ml syringe. The syringe was sealed with parafilm and placed on ice for 10 min. The cell-containing agarose plug was extruded into 10 ml of Lysis Buffer (10mM Tris pH 8.0, 50 mM NaCl, 0.1 M EDTA, 1 % Sarkosyl, 0.2 % sodium deoxycholate, a mg/ml lysozyme) and incubated at 37˚C for one hour. The agarose plug was then transferred to 40 mis of ESP Buffer (1 % Sarkosyl, 1 mg/ml proteinase-K, in 0.5M EDTA), and incubated at 55˚C for 16 hours. The solution was decanted and replaced with fresh ESP Buffer, and incubated at 55˚C for an additional hour. The agarose plugs were then placed in 50 mM EDTA and stored at 4˚C shipboard for the duration of the oceanographic cruise.

One slice of an agarose plug (72 μl) prepared from a sample collected off the Oregon coast was dialyzed overnight at 4˚C against 1 mL of buffer A (100mM NaCl, 10mM Bis Tris Propane-HCl, 100 μg/ml acetylated BSA: pH 7.0 @ 25˚C) in a 2 mL microcentrifuge tube. The solution was replaced with 250 μl of fresh buffer A containing 10 mM $MgCl_2$ and 1 mM DTT and incubated on a rocking platform for I hr at room temperature. The solution was then changed to 250 μl of the same buffer containing 4U of Sau3A1 (NEB), equilibrated to 37˚C in a water bath, and then incubated on a rocking platform in a 37˚C incubator for 45 min. The plug was transferred to a 1.5 ml microcentrifuge tube and incubated at 68˚C for 30 min to inactivate the protein. *e.g.* enzyme, and to melt the agarose. The agarose was digested and the DNA dephosphorylased using Gelase and HK-phosphatase (Epicentre), respectively, according to the manufacturer's recommendations. Protein was removed by gentle phenol/chloroform extraction and the DNA was ethanol precipitated, pelleted, and then washed with 70% ethanol. This partially digested DNA was resuspended in sterile $H_2O$ to a concentration of 2.5 ng/μl for ligation to the pFOS1 vector.

PCR amplification results from several of the agarose plugs (data not shown) indicated the presence of significant amounts of archaeal DNA. Quantitative hybridization experiments using rRNA extracted from one sample, collected at 200 m of depth off the Oregon Coast, indicated that planktonic archaea in (this assemblage comprised approximately 4.7% of the total picoplankton biomass (this sample corresponds to "PACI"-200 m in Table 1 of DeLong et al., high abundance of Archaea in Antarctic marine picoplanktion, Nature, 371:695-698, 1994). Results from archaeal-biased rDNA PCR amplification performed on agarose plug lysates confirmed the presence of relatively large amounts of archaeal DNA in this sample. Agarose plugs prepared from this picoplankton sample were chosen for subsequent fosmid library preparation. Each 1 ml agarose plug from this site contained approximately 7.5 x $10^5$ cells, therefore approximately 5.4 x. $10^5$ cells were present in the 72 μl slice used in the preparation of the partially digested DNA.

Vector arms were prepared from pFOS1 as described (Kim et al., Stable propagation of casmid sized human DNA inserts in an F factor based vector, Nucl. Acids Res., 20:10832-10835. 1992). Briefly, the plasmid was completely digested with AstII, dephosphorylated with HK phosphatase, and then digested with BamHI to generate two arms, each of which contained a *cos* site in the proper orientation for cloning and packaging ligated DNA between 35-45 kbp. The partially digested picoplankton DNA was ligated overnight to the PFOS1 arms in a 15 μl ligation reaction containing 25 ng each of vector and insert and I U of T4 DNA ligase (Boehringer-Mannheim). The ligated DNA in four microliters of this reaction was *in vitro* packaged using the Gigapack XL packaging system (Stratagene), the fosmid particles transfected to *E. coli* strain DH10B (BRL), and the cells spread onto $LB_{cm15}$ plates. The resultant fosmid clones were picked into 96-well microliter dishes containing $LB_{cm15}$ supplemented with 7% glycerol. Recombinant fosmids, each containing ca. 40 kb of picoplankton DNA insert, yielded a library of 3.552 fosmid clones, containing approximately 1.4 x $10^8$ base pairs of cloned DNA. All of the clones examined contained inserts ranging from 38 to 42 kbp. This library was stored frozen

at -80°C for later analysis.

**Example 4**

**Enzymatic Activity Assay**

The following is a representative example of a procedure for screening an expression library prepared in accordance with Example 2. In the following, the chemical characteristic Tiers are as follows:

Tier 1: Hydrolase
Tier 2: Amide. Ester and Acetal
Tier 3: Divisions and subdivisions are based upon the differences between individual substrates which are covalently attached to the functionality of Tier 2 undergoing reaction; as well as substrate specificity.
Tier 4: The two possible enantiomeric products which the enzyme may produce from a substrate.

Although the following example is specifically directed to the above mentioned tiers, the general procedures for testing for various chemical characteristics is generally applicable to substrates other than those specifically referred to in this Example.

Screening for Tier 1-hydrolase; Tier 2-amide.

The eleven plates of the Source Library were used to multiply inoculate a single plate (the "Condensed Plate") containing in each well 200 μL of LB Amp/Meth, glycerol. This step was performed using the High Density Replicating Tool (HDRT) of the Beckman Biomek with a 1 % bleach, water, isopropanol, air-dry sterilization cycle in between each inoculation. Each well of the Condensed Plate thus contained 11 different pBluescript clones from each of the eleven source library plates. The Condensed Plate was grown for 2h at 37°C and then used to inoculate two white 96-well Dynatech microtiter daughter plates containing in each well 250 μL of LB Amp/Meth, glycerol. The original condensed plates was incubated at 37°C for 18h then stored at -80°C. The two condensed daughter plates were incubated at 37°C also for 18 h. The condensed daughter plates were then heated at 70°C for 45 min. to kill the cells and inactivate the host E.coli enzymes. A stock solution of 5mg/mL morphourea phenylalanyl-7-amino-4-trifluoromethyl coumarin (Mu-PheAFC, the 'substrate') in DMSO was diluted to 600 μM with 50 mM pH 7.5 Hepes buffer containing 0.6 mg/mL of the detergent dodecyl maltoside.

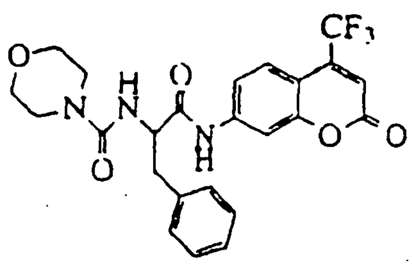

MuPheAFC

Fifty μL of the 600 μM MuPheAFC solution was added to each of the wells of the white condensed plates with one 100 μL mix cycle using the Biomek to yield a final concentration of substrate of ~ 100 μM. The fluorescence values were recorded (excitation = 400 nm, emission = 505 nm) on a plate reading fluorometer immediately after addition of the substrate (t=0). The plate was incubated at 70°C for 100 min, then allowed to cool to ambient temperature for 15 additional minutes.
The fluorescence values were recorded again (t=100). The values at t=0 were subtracted from the values at t = 100 to determine if an active clone was present.
These data indicated that one of the eleven clones in well G8 was hydrolyzing the substrate. In order to determine the individual clone which carried the activity, the eleven source library plates were thawed and the individual

clones used to singly inoculate a new plate containing LB Amp/Meth, glycerol. As above, the plate was incubated at 37°C to grow the cells, heated at 70°C to inactivate the host enzymes, and 50 μL of 600 μM MuPheAFC added using the Biomek. Additionally three other substrates were tested. The methyl umbelliferone heptanoate. the CBZ-arginine rhodamine derivative, and fluorescein-conjugated casein ( ~ 3.2 mol fluorescein per mol of casein).

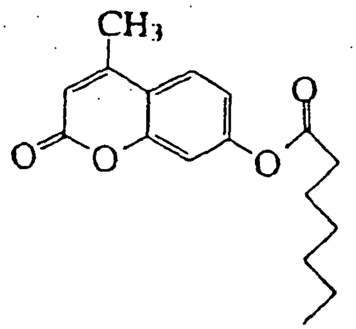

methyl umbelliferone heptanoate

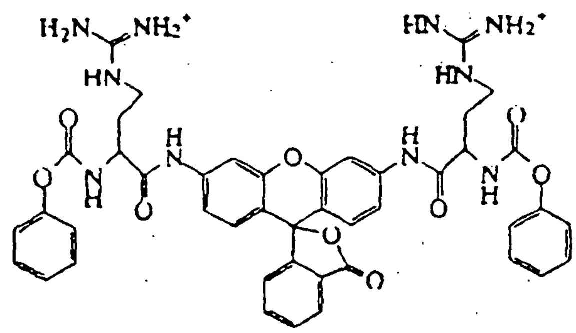

(CBZ-arginine)₁ rhodamine 110

The umbelliferone and rhodamine were added as 600 μM stock solutions in 50 μL of Hepes buffer. The fluorescein conjugated casein was also added in 50 μL at a stock concentration of 20 and 200 mg/mL. After addition of the substrates the t = 0 fluorescence values were recorded, the plate incubated at 70°C, and the t = 100 min. values recorded as above.

These data indicated that the active clone was in plate 2. The arginine rhodamine derivative was also turned over by this activity, but the lipase substrate, methyl umbelliferone heptanoate, and protein, fluorescein-conjugated casein, did not function as substrates.

Based on the above data the Tier 1 classification is 'hydrolase' and the Tier 2 classification is amide bond. There is no cross reactivity with the Tier 2-ester classification.

As shown in Figure 2, a recombinant clone from the library which has been characterized in Tier 1 as hydrolase and in Tier 2 as amide may then be tested in Tier 3 for various specificities. In Figure 2, the various classes of Tier 3 are followed by a parenthetical code which identifies the substrates of Table 1 which are used in identifying such specificities of Tier 3.

As shown in Figures 3 and 4. a recombinant clone from the library which has been characterized in Tier I as hydrolase and in Tier 2 as ester may then be tested in Tier 3 for various specificities. In Figures 3 and 4, the various classes of Tier 3 are followed by a parenthetical code which identifies the substrates of Tables 2 and 2 which are used in identifying such specificities of Tier 3. In Figures 3 and 4, $R_2$ represents the alcohol portion of the ester and $R_1$ represents the acid portion of the ester.

As shown in Figure 5, a recombinant clone from the library which has been characterized in Tier 1 as hydrolase and in Tier 2 as acetal may then be tested in Tier 3 for various specificities. In Figure 5, the various classes of Tier 3 are followed by a parenthetical code which identifies the substrates of Table 4 which are used in identifying such specificities of Tier 3.

Enzymes may be classified in Tier 4 for the chirality of the product(s) produced by the enzyme. For example, chiral amino esters may be determined using at least the following substrates:

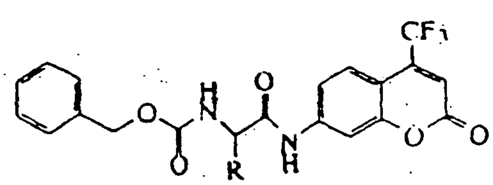

R = $CH_3$
$CH_2$-OH
$CH_2$-$CO_2^-$

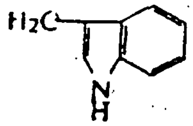

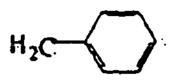

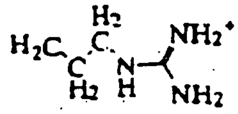

For each substrate which is turned over the enantioselectivity value, E; is determined according to the equation below:

$$E = \frac{\ln[(1-c(1+ee_p)]}{\ln[(1-c(1-ee_p)]}$$

where $ee_P$ = the enantiomeric excess (ee) of the hydrolyzed product and c = the percent conversion of the reaction. See Wong and Whitesides. Enzymes in Synthetic Organic Chemistry, 1994, Elsevier, Tarrytown, NY, pgs. 9-12.

The enantiomeric excess is determined by either chiral high performance liquid chromatography (HPLC) or chiral capillary electrophoresis (CE). Assays are performed as follows: two hundred μL of the appropriate buffer is added to each well of a 96-well white microtiter plate, followed by 50 μL of partially or completely purified enzyme solution; 50 μL of substrate is added and the increase in fluorescence monitored versus time until 50% of the substrate is consumed or the reaction stops, which ever comes first.

Enantioselectivity was determined for one of the esterases identified as follows. For the reaction to form (transesterification) or breakdown (hydrolysis) α-methyl benzyl acetate, the enantioselectivity of the enzyme was obtained by determining: $ee_c$ (the enantiomeric excess (ee) of the unreacted substrate), $ee_p$ (the ee of the hydrolyzed product), and c (the percent conversion of the reaction). The enantiomeric excess was by determined chiral high performance gas chromatography (GC). Chromatography conditions were as follows:

Sample Preparation: Samples were filtered through a 0.2 μm, 13 mm diameter PTFE filter.
Column: Supelco β-DEX 120, 0.25 mm ID. 30 m, 0.25 μm $d_f$.
Oven: 90˚C for 1 min, then 90˚C to 150˚C at 5˚C/min.
Carrier Gas: Helium, 1 mL/min for 2 min then 1 mL/min. to 3 mL/min at 0.2 mL/min.
Detector: FID. 300˚C.
Injection: 1 μL (1 mM substrate in reaction solvent), split (1:75). 200˚C.

The transesterification reaction was performed according to the procedure described in: Organic solvent tolerance. Water immiscible solvents. See below.

Transesterification with Enzyme ESL-001-01 gave the following results:

| Solvent | $\%ee_S$ | $\%ee_P$ | %c |
|---|---|---|---|
| n-heptane | 10.9 | 44.3 | 19.8 |
| toluene | 3.2 | 100 | 3.1 |

The hydrolysis reaction was performed as follows: Fifty μL of a 10 mM solution of α-methyl benzyl acetate in 10% aqueous DMSO (v/v) was added to 200 μL of 100 mM, pH 6.9 phosphate buffer. To this solution was added 250 μL of Enzyme ESL-001-01 (2 mg/mL in 100 mM, pH 6.9 phosphate buffer) and the reaction heated at 70˚C for 15 min. The reaction was worked up according to the following procedure: remove 250 μL of hydrolysis reaction mixture and add to a 1 mL Eppendorf tube. Add 250 μL of ethyl acetate and shake vigorously for 30 seconds. Allow phases to separate for 15 minutes. Pipette off 200 μL of top organic phase and filter through a 0.2 μm. 4 mm diameter PTFE filter. Analyze by chiral GC as above.

Hydrolysis with Enzyme ESL-001-01 gave the following results:

| $\%ee_S$ | $\%ee_P$ | %c |
|---|---|---|
| 100 | 0.7 | 99.3 |

## Example 5

## Testing for Physical Characteristics of a Recombinant Clone

This example describes procedures for testing for certain physical characteristics of a recombinant clone of a library.

pH optima.

Two hundred μL of 4-methyl-umbelliferyl-2,2-dimethyl-4-pentenoate was added to each well of a 96-well microtiter plate and serially diluted from column 1 to 12. Fifty μL of the appropriate 5X pH buffer was added to each row of the plate so that reaction rate in eight different pH's were tested on a single plate. Twenty μL of Enzyme ESL-001-O1 (1:3000 dilution of a 1 mg/mL stock solution) was added to each well to initiate the reaction. The increase in absorbance at 370 nm at 70˚C was monitored to determine the rate of reaction; the rate versus substrate concentration fit to the Michaelis-Menten equation to determine $V_{MAX}$ at each pH.

Enzyme ESL-001-01 gave the results shown in Figure 6.

Temperature optima:

To a one mL thermostatted cuvette was added 930 μL of 50 mM, pH 7.5 Hepes buffer. After temperature equilibration 50 μL of Enzyme ESL-001-O1 (1:8000 dilution of a 1 mg/mL stock solution in Hepes buffer) and 20 μL of 5 mM 4-methyl-umbelliferyl-heptanoate containing 30 mg/mL dodecyl maltoside. The rate of increase in absorbance at 370 nm was measured at 10, 20, 30. 40, 50, 60, 70, 80, and 90˚C.

Enzyme ESL-001-01 gave the results shown in Figure 7.

Temperature stability.

One mL samples of Enzyme ESL-001-01 (1:4000 dilution of a 1 mg/mL stock solution in Hepes buffer) were incubated at 70, 80, and 90˚C. At selected time points 25 μL aliquots were removed and assayed as above in a 96 well microtiter plate with 200 μL of 100 μM 4-methylumbelliferyl palmitate and 0.6 mg/mL dodecyl maltoside. This data was used to determine the half life for inactivation of the enzyme.

Enzyme ESL-001-01 gave the following results:

| Temperature | Half Life |
|---|---|
| 90 | 23 min. |
| 80 | 32 min. |
| 70 | 110 h |

Organic solvent tolerance.

Water miscible solvents (dimethylsulfoxide (DMSO) and tetrahydro furan (THF)).

Thirty μL of 1 mM 4-methyl-umbelliferyl-butyrate in the organic solvent was added to the wells of a 96-well microtiter plate. Two hundred forty μL of buffer and organic solvent mixture (see table below) were added the wells of the plate, followed by 30 μL of an Enzyme ESL-001-01 (1:50,000 dilution of a 1 mg/mL stock solution in 50 mM, pH 6.9 MOPS buffer) and incubation at 70˚C. The increase in fluorescence (EX = 360 nm, EM = 440 nm) was monitored versus time to determine the relative activities.

| μL Organic Solvent | μL Buffer | % Organic Solvent Final |
|---|---|---|
| 240 | 0 | 90 |
| 195 | 45 | 75 |
| 150 | 90 | 60 |
| 120 | 120 | 50 |
| 90 | 150 | 40 |
| 60 | 180 | 30 |
| 30 | 210 | 20 |
| 0 | 240 | 10 |

Enzyme ESL-001-01 OI gave the results shown in Figure 8.

Water immiscible solvents (*n*-heptane, toluene)

One mL of the solvent was added to a vial containing 1 mg of lyophilized Enzyme ESL-001-01 and a stir bar. Ten µL of 100 mM 1-phenethyl alcohol and 10 µL of 100 mM vinyl acetate were added to the vial and the vial stirred in a heating block at 70˚C for 24 h. The sample was filtered through a 0.2 µm, 4 mm diameter PTFE filter and analyzed by chiral GC as above. See previous section for data.

Specific Activity.

The specific activity was determined using 100 µM 4-methyl umbelliferyl heptanoate at 90˚C in pH 6.9 MOPS buffer. The specific activity obtained for Enzyme ESL-001-01 was 1662 µmol/min·mg

## Example 6

### Testing for Substrate Specificity of a Recombinant Clone

This example describes procedures for testing for substrate specificity of a recombinant clone of a library.

Substrate Fingerprint.

One and one quarter millimolar solutions containing 1 mg/mL of dodecyl maltoside in 50 mM pH 6.9 MOPS buffer of each of the following substrates were prepared:

4-methyl umbelliferyl acetate (A)
4-methyl umbelliferyl propanoate (B)
4-methyl umbelliferyl butyrate (C)
4-methyl umbelliferyl heptanoate (D)
4-methyl umbelliferyl α-methyl butyrate (E)
4-methyl umbelliferyl β-methylcrotonoate (F)
4-methyl umbelliferyl 2,2-dimethyl-4-pentenoate (G)
4-methyl umbelliferyl adipic acid monoester (H)
4-methyl umbelliferyl 1,4-cycylohexane dicarboxylate (I)
4-methyl umbelliferyl benzoate (M)
4-methyl umbelliferyl p-trimethyl ammonium cinnamate (N)
4-methyl umbelliferyl 4-guanidinobenzoate (O)
4-methyl umbelliferyl α-methyl phenyl acetate (P)
4-methyl umbelliferyl α-methoxy phenyl acetate (Q)
4-methyl umbelliferyl palmitate (S)
4-methyl umbelliferyl stearate (T)
4-methyl umbelliferyl oleate (U)
4-methyl umbelliferyl elaidate (W).

Two hundred µL of each of the above solutions were added to the wells of a 96 well microtiter plate, followed by 50 µL of Enzyme ESL-001-01 (1:2000 dilution of a 1 mg/mL stock solution in MOPS buffer) and incubation at 70˚C for 20 min. The fluorescence (EX = 360 nm, EM = 440 nm) was measured and fluorescence due to nonenzymatic hydrolysis was subtracted. Table 5 shows the relative fluorescence of each of the above substrates.

Numerous modifications and variations of the present invention are possible in light of the above teachings: therefore, within the scope of the claims, the invention may be practiced other than as particularly described.

**Table 1**

A2

Fluorescein conjugated casein (3.2 mol fluorescein/mol casein)
CBZ-Ala-AMC
t-BOC-Als-Ala-Asp-AMC
succinyl-Ala-Gly-Leu-AMC
CBZ-Arg-AMC
CBZ-Met-AMC
morphoures-Phe-AMC
t-BOC = t-butoxy carbonyl. CBZ = carbonyl benzyloxy.
AMC = 7-amino-4-methyl coumarin

AA3

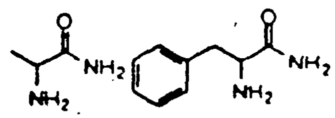

AB3

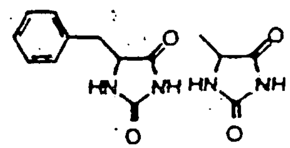

AC3

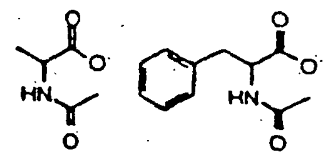

AD3

Fluorescein conjugated casein
t-BOC- Ala-Als-Asp-AFC
CBZ- Ala-Als-Lys-AFC
succinyl-Ala-Als-Phe-AFC
succinyl-Ala-Gly-Leu-AFC
AFC = 7-amino-4-trifluoromethyl coumarin.)

AE3

Fluorescein conjugated casein

AH3

succinyl-Als-Ala-Phe-AFC
CBZ-Phe-AFC
CBZ-Trp-AFC

AF3

t-BOC- Ala-Als-Asp-AFC
CBZ-Asp-AFC

A13

succinyl-Ala-Gly-Leu-AFC

(continued)

| | |
|---|---|
| | CBZ-Ala-AFC |
| AG3 | CBZ-Sewr-AFC |
| CBZ- Ala-Als-Lys-AFC | |
| CBZ-Arg-AFC | |

# Table 2

L2

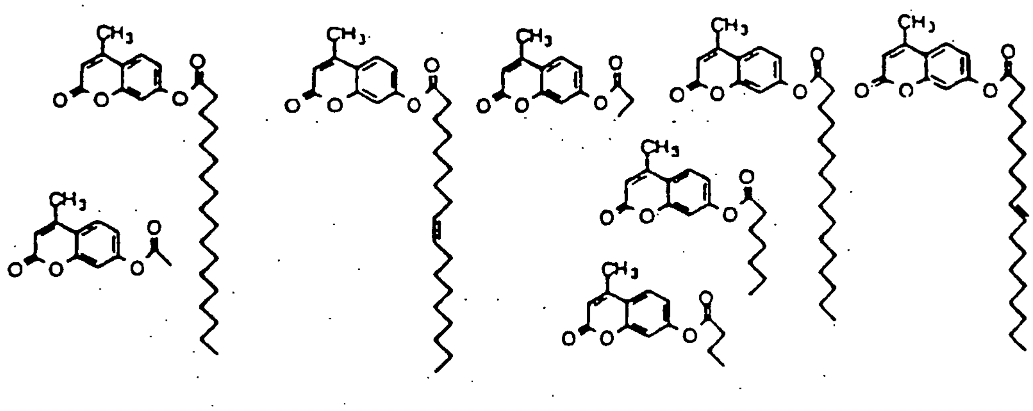

LA3

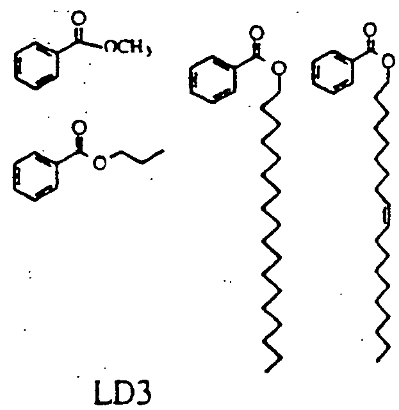

LB3

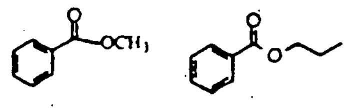

LC3

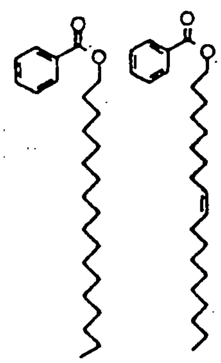

LD3

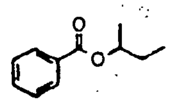

LF3

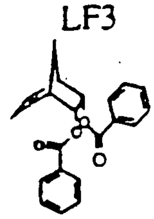

LE3

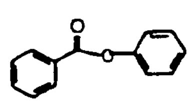

And all of L2.

LG3

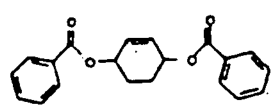

cis

# Table 3

LH3

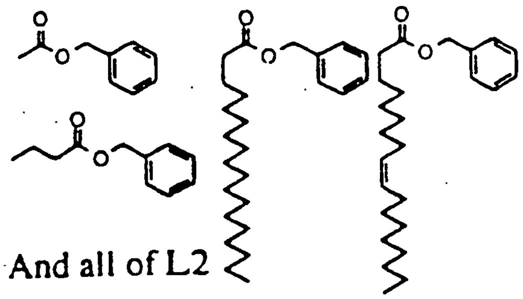

And all of L2

LI3

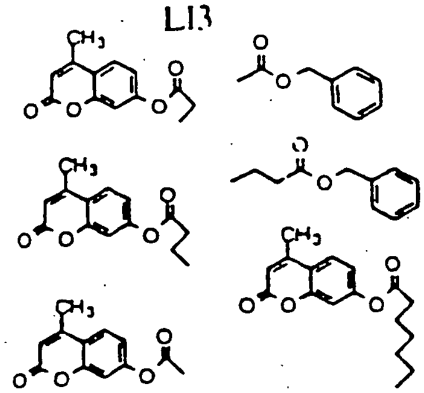

LJ3

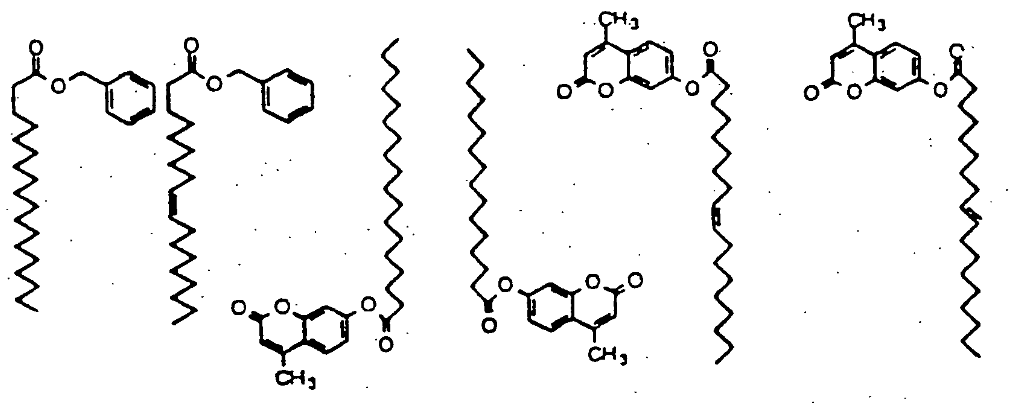

LK3

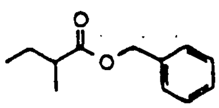

LM3

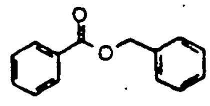

LL3

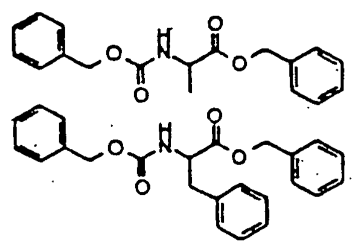

LN3

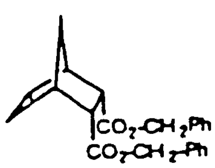

LO3

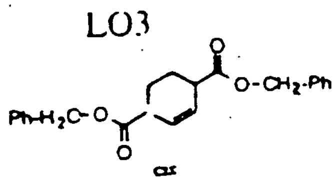

cis

**Table 4**

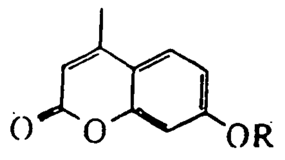

4-methyl umbelliferone
wherein R=

| | |
|---|---|
| G2 | β-D-galactose |
| | β-D-glucose |
| | β-D-glucuronide |
| GB3 | β-D-cellotrioside |
| | β-B-cellobiopyranoside |
| GC3 | β-D-galactose |
| | α-D-galactose |
| GD3 | β-D-glucose |
| | α-D-glucose |
| GE3 | β-D-glucuronide |
| GI3 | β-D-N,N-diacetylchitobiose |
| GJ3 | β-D-fucose |
| | α-L-fucose |
| | β-L-fucose |
| GK3 | β-D-mannose |
| | α-D-mannose |
| | non-Umbelliferyl substrates |
| GA3 | amylose [polyglucan α1,4 linkages], amylopectin [polyglucan branching α1,6 linkages] |
| GF3 | xylan [poly 1,4-D-xylan] |
| GG3 | amylopectin, pullulan |
| GH3 | sucrose, fructofuranoside |

Table 5

| COMPOUND | RELATIVE FLUORESCENCE |
|---|---|
| A | 60.6 |
| B | 73.6 |
| C | 100.0 |
| D | 84.2 |
| E | 29.1 |
| F | 5.4 |
| G | 7.1 |
| H | 0.9 |
| I | 0.0 |
| M | 9.4 |
| N | 0.5 |
| O | 0.5 |
| P | 4.0 |

(continued)

| COMPOUND | RELATIVE FLUORESCENCE |
|---|---|
| Q | 11.3 |
| S | 0.6 |
| T | 0.1 |
| U | 0.3 |
| W | 0.2 |

## Claims

1. A process of screening clones having DNA from an uncultivated microorganism for a specified protein activity, which process comprises:

   screening for a specified protein activity in a library of clones prepared by

   (i) recovering DNA from a DNA population derived from at least one uncultivated microorganism; and
   (ii) transforming a host with recovered DNA to produce a library of clones which is screened for the specified protein activity.

2. The process of claim 1 wherein the recovered DNA is amplified.

3. The process of claim 1 wherein the recovered DNA is ligated into a vector.

4. The process of any one of claims 1 to 3, wherein the protein is an enzyme.

5. The process of claim 4 wherein the vector into which the recovered DNA is ligated comprises at least one DNA sequence capable of regulating production of a detectable enzyme activity from said recovered DNA.

6. The process of claims 3 or 5 wherein the vector into which the recovered DNA has been ligated is used to transform a host.

## Patentansprüche

1. Verfahren zum Screenen von Klonen mit DNA von einem nicht-gezüchteten Mikroorganismus auf eine bestimmte Proteinaktivität, wobei das Verfahren umfasst:

   Screenen auf eine bestimmte Proteinaktivität in einer Bibliothek von Klonen, die hergestellt wurde durch

   (i) Gewinnung von DNA aus einer DNA-Population, die aus mindestens einem nicht-gezüchteten Mikroorganismus abgeleitet ist, und
   (ii) Transformation eines Wirts mit gewonnener DNA, um eine Bibliothek von Klonen herzustellen, die auf die bestimmte Proteinaktivität gescreent wird.

2. Verfahren nach Anspruch 1, wobei die gewonnene DNA amplifiziert wird.

3. Verfahren nach Anspruch 1, wobei die gewonnene DNA in einen Vektor ligiert wird.

4. Verfahren nach einem der Ansprüche 1 bis 3, wobei das Protein ein Enzym ist.

5. Verfahren nach Anspruch 4, wobei der Vektor, in den die gewonnene DNA ligiert ist, mindestens eine DNA-Sequenz umfasst, die fähig ist, die Produktion einer nachweisbaren Enzymaktivität von der gewonnenen DNA zu regulieren.

6. Verfahren nach Anspruch 3 oder 5, wobei der Vektor, in den die gewonnene DNA ligiert wurde, verwendet wird,

um einen Wirt zu transformieren.

**Revendications**

1. Procédé pour cribler des clones ayant un ADN provenant d'un micro-organisme non cultivé pour une activité protéique spécifique, ledit procédé comprenant :

   le criblage pour une activité protéique spécifique d'une banque de clones préparée par

   (i) l'extraction d'ADN à partir d'une population d'ADN dérivée d'au moins un micro-organisme non cultivé ; et
   (ii) la transformation d'un hôte avec l'ADN extrait pour produire une banque de clones qui est criblée pour l'activité protéique spécifique.

2. Procédé selon la revendication 1, dans lequel l'ADN extrait est amplifié.

3. Procédé selon la revendication 1, dans lequel l'ADN extrait est lié dans un vecteur.

4. Procédé selon l'une quelconque des revendications 1 à 3, dans lequel la protéine est une enzyme.

5. Procédé selon la revendication 4, dans lequel le vecteur dans lequel l'ADN extrait est lié comprend au moins une séquence d'ADN capable de réguler la production d'une activité enzymatique détectable à partir dudit ADN extrait.

6. Procédé selon les revendications 3 ou 5, dans lequel le vecteur dans lequel l'ADN extrait a été lié est utilisé pour transformer un hôte.

Figure 1

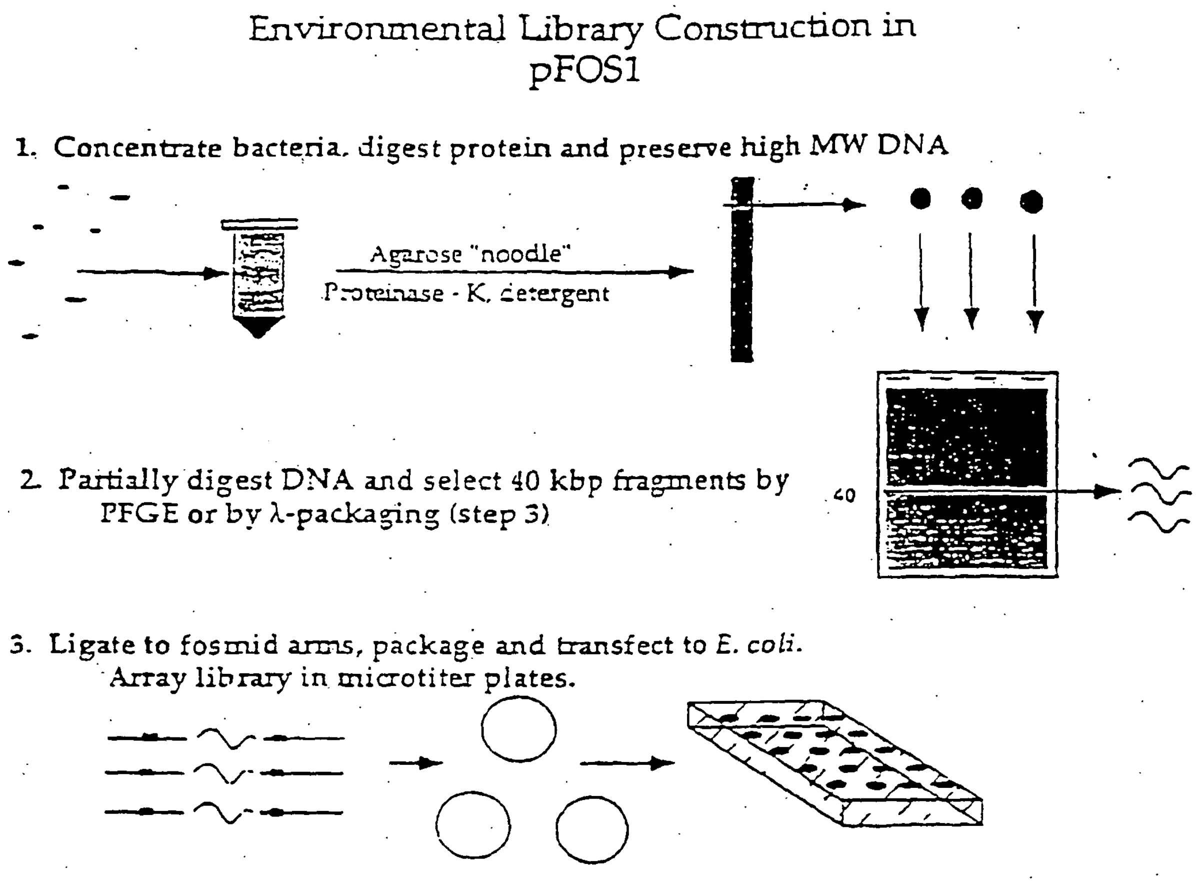
Environmental Library Construction in pFOS1
1. Concentrate bacteria, digest protein and preserve high MW DNA
Agarose "noodle"
Proteinase - K, detergent
2. Partially digest DNA and select 40 kbp fragments by PFGE or by λ-packaging (step 3)
40
3. Ligate to fosmid arms, package and transfect to E. coli. Array library in microtiter plates.

Figure 2

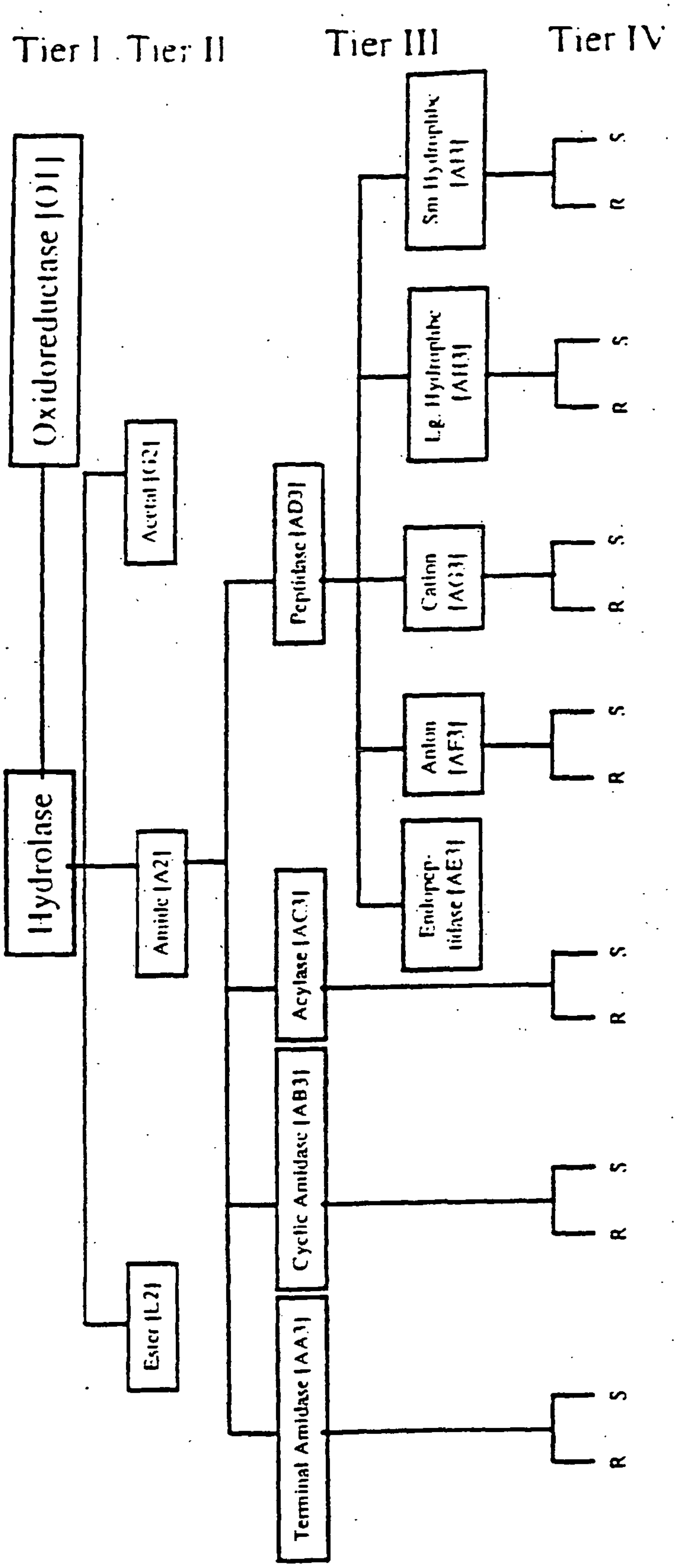
Tier I
Tier II
Tier III
Tier IV
Oxidoreductase [O1]
Hydrolase
Acetal [G2]
Amide [A2]
Ester [L2]
Peptidase [AD3]
Acylase [AC3]
Cyclic Amidase [AB3]
Terminal Amidase [AA3]
Sm Hydrophbc
Lg. Hydrophbc
Cation [AG3]
Anion [AF3]
Endopeptidase [AE3]
R
S

Figure 3

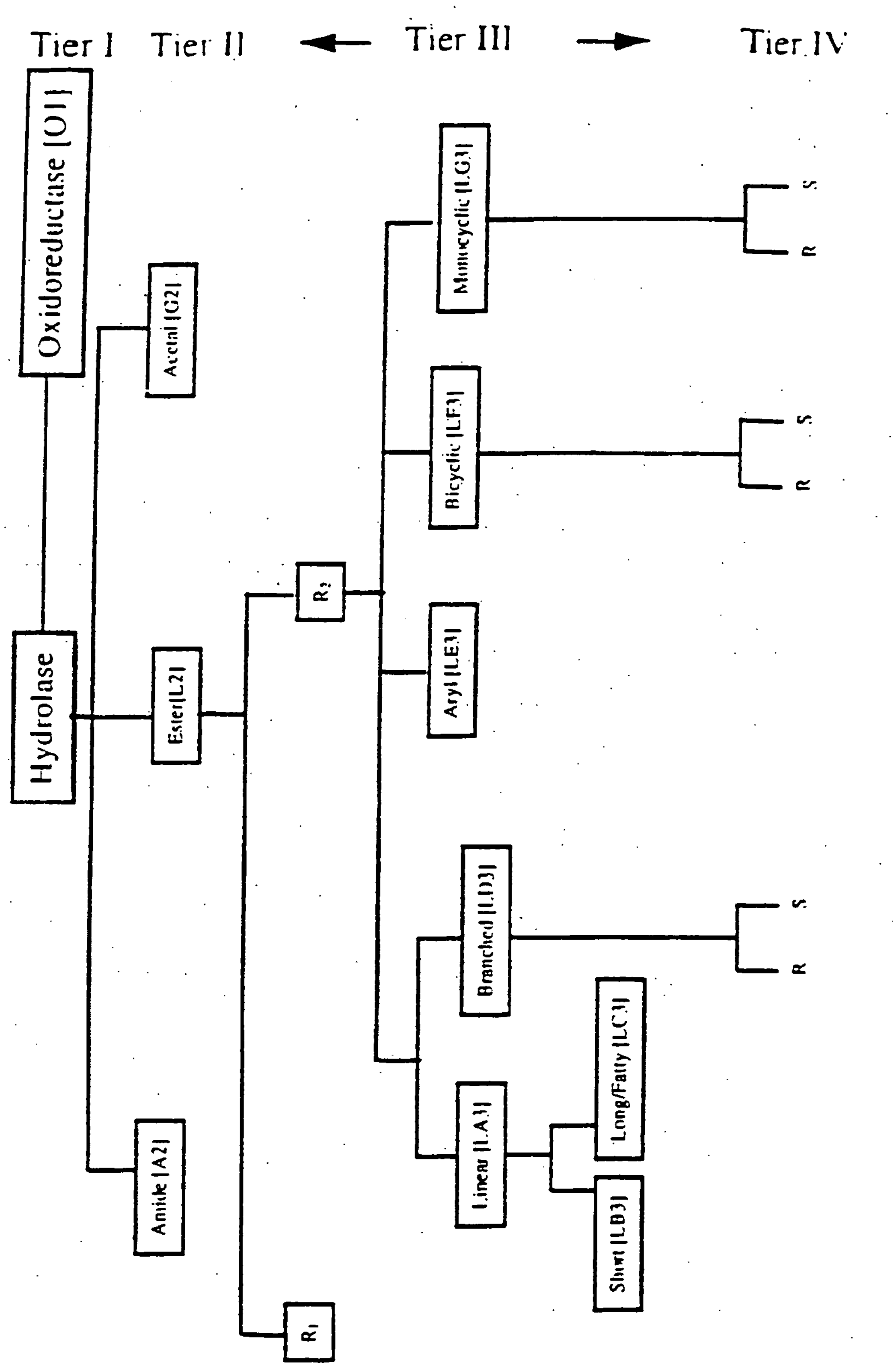

Tier I
Tier II
Tier III
Tier IV
Oxidoreductase [O1]
Hydrolase
Acetal [G2]
Ester [L2]
Amide [A2]
R2
R1
Monocyclic [LG3]
Bicyclic [LF3]
Aryl [LE3]
Branched [LD3]
Linear [LA3]
Long/Fatty [LC3]
Short [LB3]
R
S
R
S
R
S

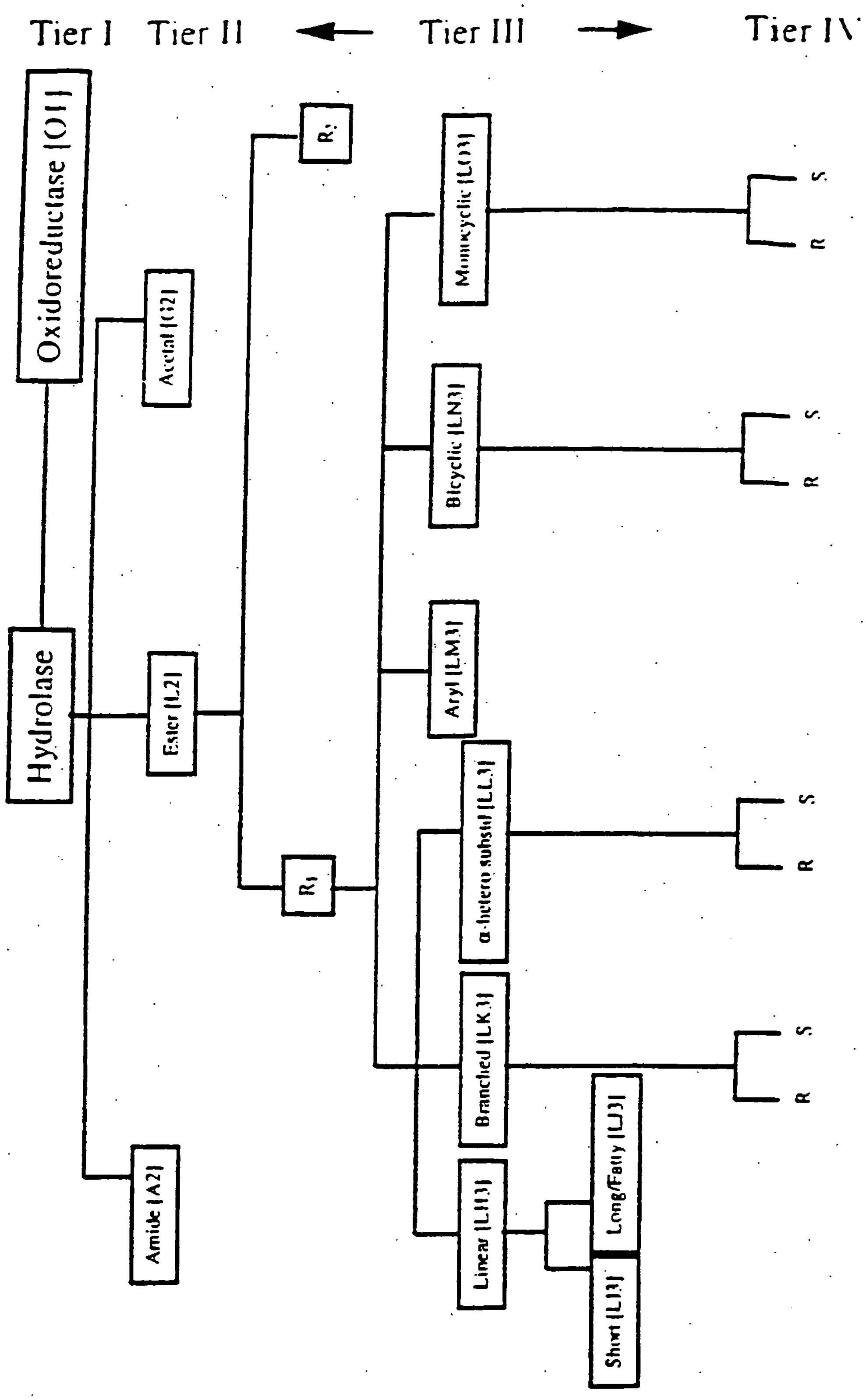

Tier I
Tier II
Tier III
Tier IV
Oxidoreductase
Hydrolase
Ester [L2]
Amide [A2]
Aryl [LM3]
Bicyclic [LN3]
Branched [LK3]
Long/Fatty [LJ3]
R
S


Figure 4

Figure 5

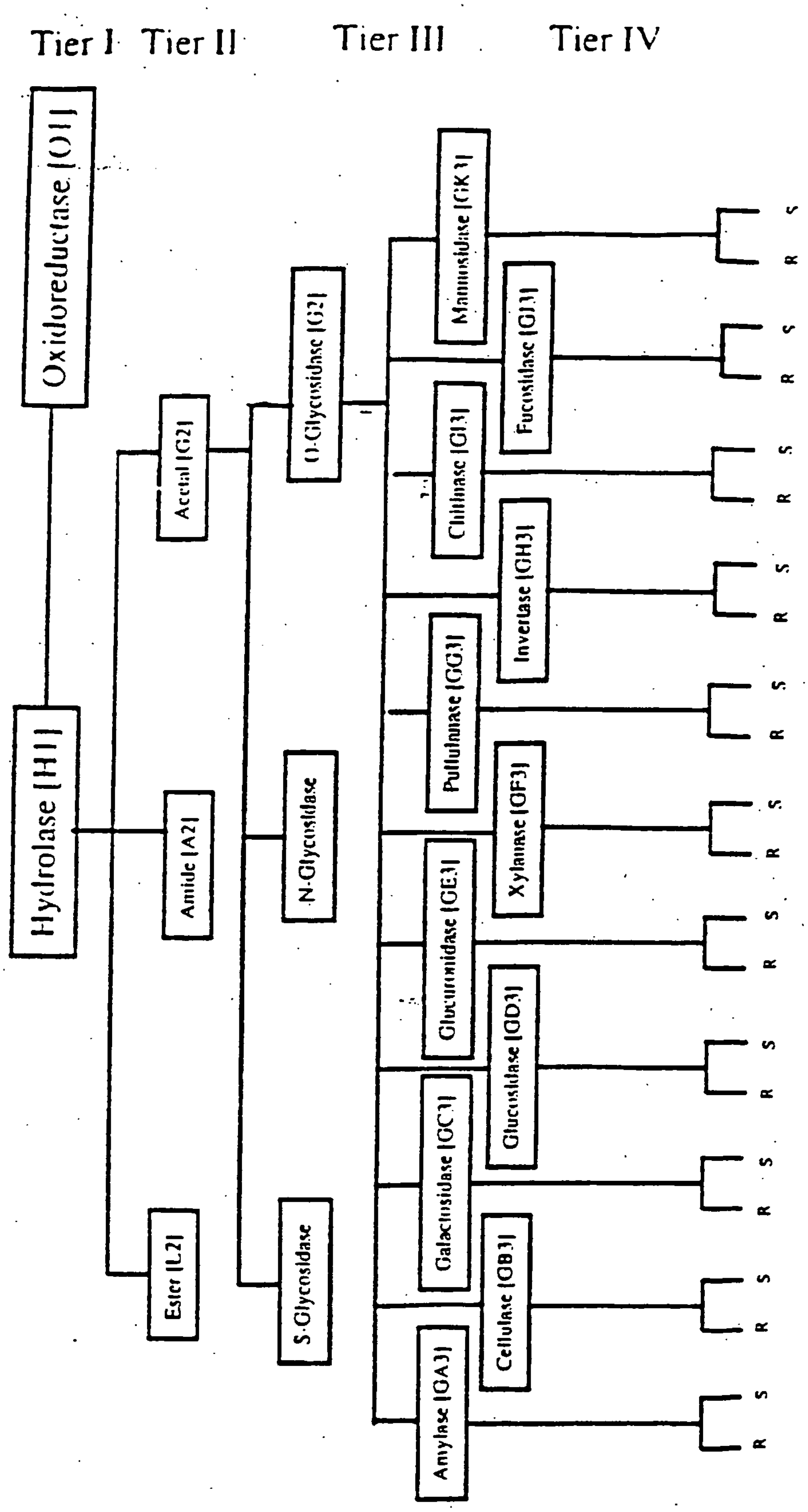
Tier I
Tier II
Tier III
Tier IV
Oxidoreductase [O1]
Hydrolase [H1]
Acetal [G2]
Amide [A2]
Ester [L2]
O-Glycosidase [G2]
N-Glycosidase
S-Glycosidase
Mannosidase [GK3]
Fucosidase [GJ3]
Chitinase [GI3]
Invertase [GH3]
Pullulanase [GG3]
Xylanase [GF3]
Glucuronidase [GE3]
Glucosidase [GD3]
Galactosidase [GC3]
Cellulase [GB3]
Amylase [GA3]
R
S

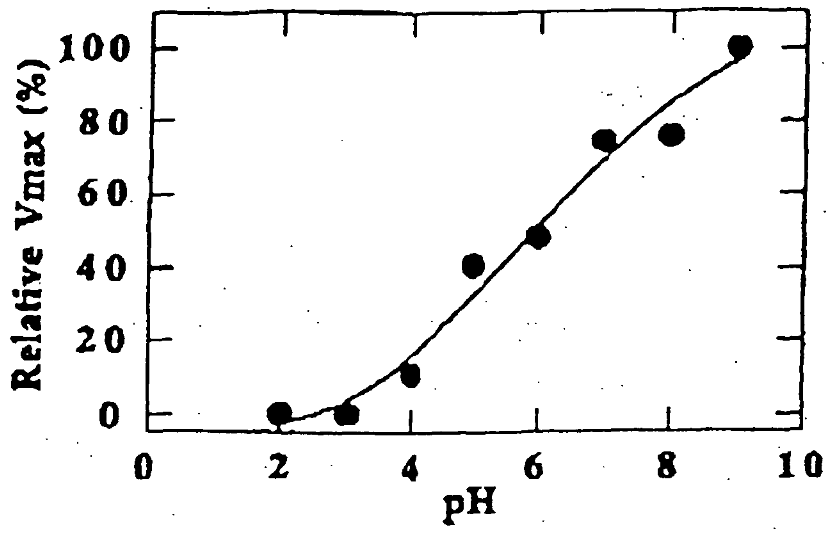


Figure 6

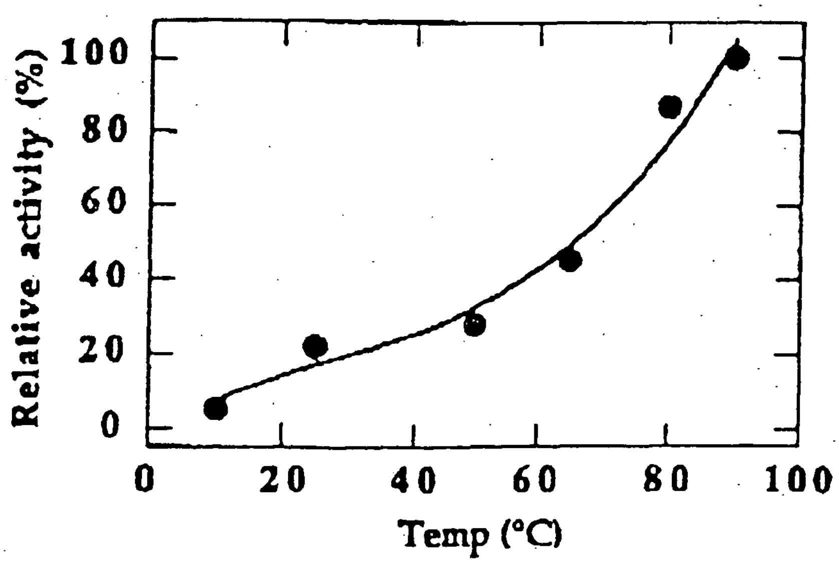


Figure 7

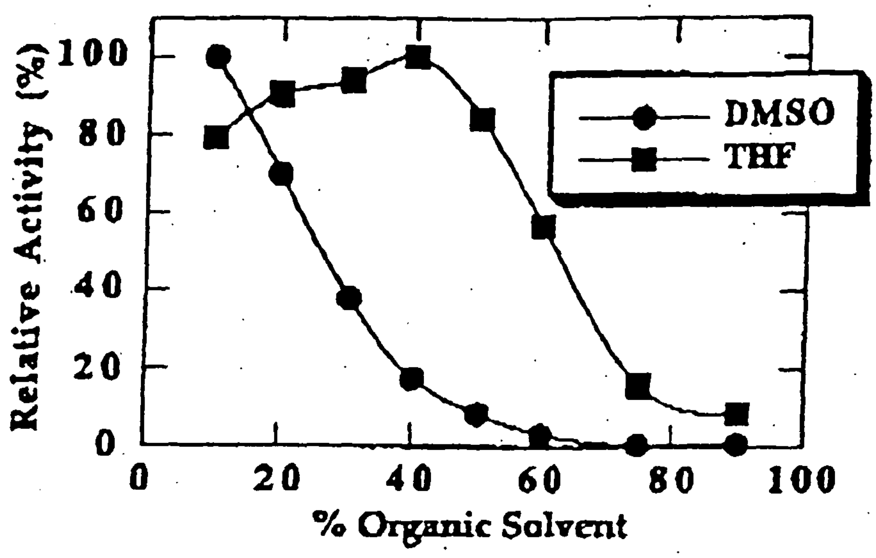
Relative Activity (%)
100
80
60
40
20
0
0
20
40
60
80
100
% Organic Solvent
DMSO
THF

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Davis, L. ; Dibner, M. ; Battey, I.** *Basic Methods in Molecular Biology,* 1996
- *Current Protocols in Molecular Biology,* 1987, vol. 2.4.1
- **Maniatis, T. ; Fritsch, E.F. ; Sambrook, J.** Molecular Cloning. Cold Spring Harbor Press, 1982
- **Hay ; Short.** *J. Strategies,* 1992, vol. 5, 16
- **DeLong et al.** high abundance of Archaea in Antarctic marine picoplanktion. *Nature,* 1994, vol. 371, 695-698
- **Kim et al.** Stable propagation of casmid sized human DNA inserts in an F factor based vector. *Nucl. Acids Res.,* 1992, vol. 20, 10832-10835
- **Wong ; Whitesides.** Enzymes in Synthetic Organic Chemistry. Elsevier, 1994, 9-12